# EUROPÄISCHE PATENTSCHRIFT

(11) **EP 1 789 015 B1**
(45) Veröffentlichungstag und Bekanntmachung des Hinweises auf die Patenterteilung: **23.04.2014**
(21) Anmeldenummer: 05772752.1
(22) Anmeldetag: 18.08.2005
(51) Int. Cl.: A61K 8/22, A61Q 5/08, A61Q 5/10

(54) **VERFAHREN ZUR MODISCHEN FARBVERÄNDERUNG KERATINHALTIGER FASERN**
METHOD FOR STYLISHLY CHANGING THE COLOR OF KERATINIC FIBERS
PROCEDE POUR CHANGER LA COULEUR DE FIBRES KERATINIQUES SELON LA MODE

(30) Priorität: 18.09.2004 DE 102004045414
(43) Veröffentlichungstag der Anmeldung: 30.05.2007
(73) Patentinhaber: Henkel AG & Co. KGaA, 40589 Düsseldorf (DE)
(72) Erfinder: SEILER, Martina, 47228 Duisburg (DE); HOLLENBERG, Detlef, 40699 Erkrath (DE)
(86) Internationale Anmeldenummer: PCT/EP2005/008949
(87) Internationale Veröffentlichungsnummer: WO 2006/029686

(56) Entgegenhaltungen:
- EP-A- 0 705 598
- WO-A-01/72272
- WO-A-2004/058202
- US-A- 5 224 964

## Beschreibung

Die vorliegende Erfindung betrifft ein Verfahren zur Färbung keratinhaltiger Fasern, insbesondere menschlicher Haare, in welchem in einem Aufhellschritt selektiv einige Fasern einer Blondierung unterworfen werden und anschließend die gesamte Menge an keratinhaltigen Fasern mit einem speziellen Färbemittel gefärbt werden. Weiterhin ist ein Kit-of-parts, enthaltend ein Aufhellmittel und ein Färbemittel, sowie Applikationshilfen Gegenstand der Erfindung und die Verwendung dieses Kits in dem erfindungsgemäßen Färbeverfahren.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle. Sieht man von den Blondiermitteln, die eine oxidative Aufhellung der Haare durch Abbau der natürlichen Haarfarbstoffe bewirken, ab, so sind im Bereich der Haarfärbung im wesentlichen vier Typen von Haarfärbemitteln von Bedeutung:
Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich zwar durch hervorragende, lang anhaltende Färbeergebnisse aus. Für natürlich wirkende Färbungen muß
aber üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet.

Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterozyklische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazolon-5, 4-Amino-3-methylphenol, 2-Aminomethyl-4-aminophenol, 2-Hydroxy-4,5,6-triaminopyrimidin, 2,4-Dihydroxy-5,6-diaminopyrimidin, 2,5,6-Triamino-4-hydroxypyrimidin und 1,3-N,N'-Bis(2-hydroxyethyl)-N,N'-bis(4-aminophenyl)-diaminopropan-2-ol.

Als Kupplerkomponenten werden in der Regel m-Phenylendiaminderivate, Naphthole, Resorcin und Resorcinderivate, Pyrazolone und m-Aminophenole verwendet. Als Kupplersubstanzen eignen sich insbesondere 1-Naphthol, 1,5- Dihydroxynaphthalin, 2,7-Dihydroxynaphthalin und 1,7-Dihydroxynaphthalin, 5-Amino-2-methylphenol, m-Aminophenol, Resorcin, Resorcinmonomethylether, m-Phenylendiamin, 1-Phenyl-3-methylpyrazolon-5, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin und 2-Methyl-4-chlor-5-aminophenol.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Haar aufziehen und keinen oxidativen Prozeß zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel deutlich empfindlicher als die oxidativen Färbungen, so daß dann sehr viel schneller eine vielfach unerwünschte Nuancenverschiebung oder gar eine sichtbare "Entfärbung" eintritt.

Schließlich hat weiteres Färbeverfahren große Beachtung gefunden. Bei diesem Verfahren werden Vorstufen des natürlichen Haarfarbstoffes Melanin auf das Haar aufgebracht; diese bilden dann im Rahmen oxidativer Prozesse im Haar naturanaloge Farbstoffe aus. Ein solches

Verfahren mit 5,6-Dihydroxyindolin als Farbstoffvorprodukt wurde in der EP-B1-530 229 beschrieben. Bei, insbesondere mehrfacher, Anwendung von Mitteln mit 5,6-Dihydroxyindolin ist es möglich, Menschen mit ergrauten Haaren die natürliche Haarfarbe wiederzugeben. Die Ausfärbung kann dabei mit Luftsauerstoff als einzigem Oxidationsmittel erfolgen, so daß auf keine weiteren Oxidationsmittel zurückgegriffen werden muß. Bei Personen mit ursprünglich mittelblondem bis braunem Haar kann das Indolin als alleinige Farbstoffvorstufe eingesetzt werden. Für die Anwendung bei Personen mit ursprünglich roter und insbesondere dunkler bis schwarzer Haarfarbe können dagegen befriedigende Ergebnisse häufig nur durch Mitverwendung weiterer Farbstoffkomponenten, insbesondere spezieller Oxidationsfarbstoffvorprodukte, erzielt werden. Eine weitere Möglichkeit keratinhaltige Fasern zu färben, bietet die Verwendung von Färbemitteln, die eine Kombination aus Komponente
- A: Verbindungen, die eine reaktive Carbonylgruppe enthalten mit Komponente
- B: Verbindungen, ausgewählt aus (a) CH-aciden Verbindungen, (b) Verbindungen mit primärer oder sekundärer Aminogruppe oder Hydroxygruppe, ausgewählt aus primären oder sekundären aromatischen Aminen, stickstoffhaltigen heterozyklischen Verbindungen und aromatischen Hydroxyverbindungen, (c) Aminosäuren, (d) aus 2 bis 9 Aminosäuren aufgebauten Oligopeptiden
enthalten. Die entsprechende Färbemethode (im folgenden Oxofärbung genannt) wird beispielsweise in den Druckschriften WO-A1-99/18916, WO-A1-00/38638, WO-A1-01/34106 und WO-A1-01/47483 beschrieben. Die resultierenden Färbungen besitzen teilweise Farbechtheiten auf der keratinhaltigen Faser, die mit denen der Oxidationsfärbung vergleichbar sind. Das mit der schonenden Oxofärbung erzielbare Nuancenspektrum ist sehr breit und die erhaltene Färbung weist oftmals eine akzeptable Brillanz und Farbtiefe auf. Die vorgenannten Komponenten A und B, im weiteren als Oxofarbstoffvorprodukte bezeichnet, sind im allgemeinen selbst keine Farbstoffe, und eignen sich daher jede für sich genommen allein nicht zur Färbung keratinhaltiger Fasern. In Kombination bilden sie in einem nichtoxidativen Prozess Farbstoffe aus. Unter Verbindungen der Komponente B können allerdings auch entsprechende Oxidationsfarbstoffvorprodukte vom Entwickler- und/oder Kupplertyp mit oder ohne Einsatz eines Oxidationsmittels Verwendung finden. Somit läßt sich die Methode der Oxofärbung ohne weiteres mit dem oxidativen Färbesystem kombinieren.

Zur Aufhellung keratinhaltiger Fasern werden sogenannte Bleichmittel verwendet. Letztere enthalten Oxidationsmittel, welche auf den natürlichen Haarfarbstoff Melanin und gegebenenfalls auf in der Faser befindliche synthetische Farbstoffe oxidativ einwirken und
dadurch eine Farbveränderung und optimalerweise eine Aufhellung der Haarfarbe verursachen. Die Grundlagen der Blondier- und oxidativen Färbeverfahren sind dem Fachmann bekannt und in einschlägigen Monographien, z.B. von K. Schrader, Grundlagen und Rezepturen der Kosmetika, 2. Auflage, 1989, Dr. Alfred Hüthig Verlag, Heidelberg, oder W. Umbach (Hrg.), Kosmetik, 2. Auflage, 1995, Georg Thieme Verlag, Stuttgart, New York, zusammenfassend beschrieben.

Allerdings ist die gezielte oxidative Aufhellung von mit Färbemitteln behandelten keratinhaltigen Fasern nicht ohne weiteres zu bewerkstelligen. Oftmals resultieren aus der oxidativen Aufhellung solcher farbveränderter Fasern unerwünschte Farbverschiebungen zu z.B. Orange- oder Grüntönen, die unbedingt vermieden werden müssen. Es sind nur solche Farbeffekte im Rahmen der Aufhellung erstrebenswert, die eine Aufhellung des Ausgangsfarbtons bedeuten. Ferner ist es erstrebenswert, einen wohldefinierten Teil des Kopfhaares wie z.B. Strähnchen gleichmäßig farblich zu nuancieren und diese wohldefinierten Teile wiederum möglichst gleichmäßig über den gesamten Kopfhaarbereich zu verteilen. Dies bedeutet für eine Strähnchennuancierung, dass eine Haarfaser gleichmäßig vom Haaransatz bis in die Spitzen nuanciert werden muss und diese Strähnchen sich möglichst gleichverteilt sowohl im Deckhaar, als auch in den darunter liegenden Haarbereichen ansiedeln sollten. Auf diese Weise werden gleichmäßige und natürlich wirkende, helle Farbreflexe erhalten.

WO 2001/072272 A offenbart Bleich- und Färbemischungen für menschliches Haar, die eine gute Lagerstabilität aufweisen und die eine stabile alkalische Oxidationslösung umfassen. US 5224964 offenbart ein Färbeverfahren zur Erzielung von Aufhellungen und Reflexen auf dem Haar, bei dem das Haar vorgebleicht und anschließend gefärbt wird, wobei nur Pigmentfarbstoffe und keine Oxidationsfarbstoffe verwendet werden sollen, da mit letzteren eine zu gleichmäßige Färbung erhalten wird.

Aufgabe der vorliegenden Erfindung ist es daher, ein Verfahren bereitzustellen, welches in einer Grundfärbung mit Färbemitteln eingefärbten keratinhaltigen Fasern über den gesamten Faserbündelbereich kontrastreiche, gleichmäßige und natürliche wirkende, helle Farbreflexe verleiht.

Es wurde überraschenderweise gefunden, dass sich gleichmäßige Farbreflexe ohne unerwünschte Farbverschiebungen bei der oxidativen Aufhellung erzielen lassen, wenn zuerst die oxidative Aufhellung erfolgt und sich dann der Färbeschritt anschließt, und in diesem Verfahren ein spezielles Färbemittel und ein Aufhellmittel, im weiteren Nuanciermittel genannt, verwendet wird.

Ein erster Gegenstand der Erfindung ist daher ein Verfahren zur Färbung keratinhaltiger Fasern, insbesondere menschlicher Haare, in welchem
- A: auf einen Teil der Fasern ein Nuanciermittel, enthaltend in einem kosmetischen Träger mindestens ein Verdickungsmittel, Wasserstoffperoxid und mindestens ein Alkalisierungsmittel, aufgetragen und nach einer Einwirkzeit Z1 wieder abgespült wird,
- B: die Fasern im Anschluß gegebenenfalls getrocknet werden und anschließend
- C: auf die gesamten Fasern ein Färbemittel aufgetragen und nach einer Einwirkzeit Z2 wieder abgespült wird,
wobei das Färbemittel als farbgebende Komponente mindestens zwei Oxidationsfarbstoffvorprodukte enthält,
dadurch gekennzeichnet, dass das Färbemittel eine Kombination aus
(a) mindestens einem p-Phenylendiaminderivat gemäß Formel (E1), 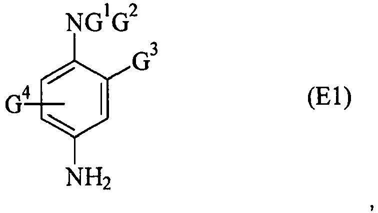 wobei
   - G¹ steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen 4'-Aminophenylrest oder einen C₁- bis C₄-Alkylrest, der mit einer stickstoffhaltigen Gruppe, einem Phenyl- oder einem 4'-Aminophenylrest substituiert ist;
   - G² steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest oder einen C₁- bis C₄-Alkylrest, der mit einer stickstoffhaltigen Gruppe substituiert ist;
   - G³ steht für ein Wasserstoffatom, ein Halogenatom, wie ein Chlor-, Brom-, lod- oder Fluoratom, einen C₁- bis C₄-Alkylrest, einen C₁- bis C₄-Monohydroxyalkylrest, einen C₂- bis C₄-Polyhydroxyalkylrest, einen C₁- bis C₄-Hydroxyalkoxyrest, einen C₁- bis C₄-Acetylaminoalkoxyrest, einen C₁-bis C₄-Mesylaminoalkoxyrest oder einen C₁- bis C₄-Carbamoylamino-alkoxyrest;
   - G⁴ steht für ein Wasserstoffatom, ein Halogenatom oder einen C₁- bis C₄-Alkylrest oder
   - wenn G³ und G⁴ in ortho-Stellung zueinander stehen, können sie gemeinsam eine verbrückende α,ω-Alkylendioxogruppe bilden,
(b) mindestens einem p-Aminophenolderivat gemäß Formel (E3), 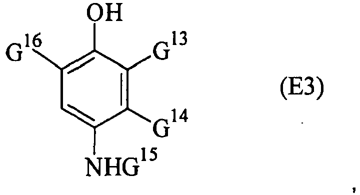 wobei:
   - G¹³ steht für ein Wasserstoffatom, ein Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest, einen Hydroxy-(C₁- bis C₄)-alkylaminorest, einen C₁- bis C₄-Hydroxyalkoxyrest, einen C₁- bis C₄-Hydroxyalkyl-(C₁-bis C₄)-aminoalkylrest oder einen (Di-C₁- bis C₄-Alkylamino)-(C₁- bis C₄)-alkylrest, und
   - G¹⁴ steht für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest oder einen C₁- bis C₄-Cyanoalkylrest,
   - G¹⁵ steht für Wasserstoff, einen C₁- bis C₄-Alkylrest, einen C₁- bis C₄-Monohydroxyalkylrest, einen C₂- bis C₄-Polyhydroxyalkylrest, einen Phenylrest oder einen Benzylrest, und
   - G¹⁶ steht für Wasserstoff oder ein Halogenatom,
(c) mindestens einem Pyridinderivat, ausgewählt aus 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Di-hydroxy-4-methylpyridin, 2,6-Diaminopyridin, 2,3-Diamino-6-methoxy-pyridin und 3,5-Diamino-2,6-dimethoxypyridin, als Kuppler, sowie
(d) mindestens einer Verbindung, ausgewählt aus m-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methyl-phenol, 5-Amino-4-methoxy-2-methylphenol, 5-(2'-Hydroxyethyl)amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol, als Kuppler enthält.

Unter keratinhaltigen Fasern werden im Rahmen dieser Anmeldung Pelze, Wolle, Federn und insbesondere menschliche Haare verstanden. Obwohl die Färbemittel des erfindungsgemäßen Verfahrens in erster Linie zum Färben von Keratinfasern geeignet sind, steht prinzipiell einer Verwendung auch auf anderen Gebieten, insbesondere in der Farbphotographie, nichts entgegen.

Es wird ein schärferer Kontrastübergang zwischen dem im Schritt C gefärbten und dem aus Schritt A oxidativ aufgehellten Haarbereichen bewirkt, wenn die Haare vor Schritt A trocken sind und das Nuanciermittel auf das trockene Haar appliziert wird.

Das Nuanciermittel wird auf einen Teil der gesamten zu behandelnden keratinhaltigen Fasern aufgetragen. Dies bedeutet, daß aus der Menge der Fasern eine oder mehrere Teilmengen als Faserbündel ausgewählt werden. Diese Faserbündel sollten, wenn es sich um menschliche Haare handelt, zur Erzielung gleichmäßig verteilter Farbeffekte nicht nur aus dem Deckhaar, sondern auch aus den unter dem Deckhaar liegenden Haarbereichen ausgewählt sein. Ferner werden die ausgewählten Faserbündel bevorzugt möglichst gleichmäßig über die Fläche des gesamten Kopfhaarbereichs selektiert. Die ausgewählten Faserbündel werden entweder vollständig oder partiell mit dem Nuanciermittel behandelt. Bei einer vollständigen Behandlung werden Strähnchen mit hellen Farbeffekten erhalten. Bei einer partiellen Behandlung lassen sich beispielsweise die Faserspitzen oder andere ausgewählte Bereiche des Faserbündels selektiv nuancieren.

Das Nuanciermittel kann prinzipiell mit den Händen unter Verwendung von entsprechend geeigneten Schutzhandschuhen auf die selektierte Fasersträhne appliziert werden. Das Nuanciermittel wird jedoch bevorzugt mit einem Applikator, wie beispielsweise einer Bürste, einem Pinsel oder einer Applicette auf die zur Nuancierung vorgesehenen Haarbereiche aufgetragen. Unter einer Applicette wird ein breiter Pinsel verstanden, an dessen Stielende sich eine Spitze befindet, die das Abteilen von Faserbündeln bzw. Strähnchen aus der Gesamtmenge der Fasern erlaubt und vereinfacht.

Es ist bevorzugt, insbesondere bei der Durchführung der Nuancierung an kurzem Haar bis 20 cm Länge oder zur Nuancierung der Haarspitzen, als Applikator eine Rundbürste zu verwenden. Eine Rundbürste besitzt einen Stiel und einen Borstenkopf. Die Borsten des Borstenkopfes sind bei einer Rundbürste in Form eines Zylinders um einen zentralen Körper des Borstenkopfes angeordnet, wobei die Summe der befestigten Borsten mit einem Borstenende eine im wesentlichen zylindrische Außenfläche beschreibt während sie mit dem anderen Ende am Körper des Borstenkopfes befestigt ist. Der Körper des Borstenkopfes ist wiederum an einem Ende des Stils der Rundbürste positioniert oder ist ein Teil eines Endbereichs des Stils. Die in dem erfindungsgemäßen Verfahren verwendete Rundbürste besitzt bevorzugt Borsten mit einer Länge von maximal 2 cm und hat einen Durchmesser des zylindrischen Borstenkopfes von maximal 2,5 cm. Der Zylinder des Borstenkopfes hat eine Länge von maximal 4 cm. Zur Auftragung des Nuanciermittels eignet sich hervorragend eine Mascarabürste.

Eine besonders bevorzugte Möglichkeit, eine verbesserte gleichmäßige Applikation des Nuanciermittels zur Applikation der Farbeffekte in Form von Strähnchen über den Kopfhaarbereich unter Erhalt scharfer Kontraste zu erzielen, bietet die Verwendung einer Haube mit einem gleichmäßig über die Oberfläche der Haube verteilten, gegebenenfalls nur vorgezeichneten, Lochraster. In einer bevorzugten Ausführungsform des Verfahrens werden daher die bevorzugt trockenen Fasern mit einer solchen Haube abgedeckt. Mit einem Hilfsmittel in Form einer Häkelnadel werden anschließend Faserbündel bzw. Haarsträhnen, durch die Löcher der Haube hindurchgezogen. Danach wird unter Benutzung geeigneter Schutzhandschuhe das Nuanciermittel gemäß Schritt A auf die ausgewählten Haarbereiche mit den Händen wie bei einem Shampoo oder mit einem Pinsel verteilt. Nach der Einwirkzeit Z1 wird das Nuanciermittel abgespült und die Haube entfernt.

Die Haube ist bevorzugt aus mindestens einer Folie gefertigt, welche bevorzugt aus Polyethylen oder Polyvinylchlorid besteht. Wenn die Haube aus einer Folienschicht besteht, ist es möglich, das Lochraster vorzustanzen oder vorzuzeichnen. Bei einem lediglich vorgezeichneten Raster besitzt die Folie keine Löcher und muß an der ausgewählten vorgezeichneten Stelle durchstoßen und die Haarsträhne anschließend durch das dabei entstandene Loch gezogen werden. Es ist erfindungsgemäß bevorzugt, die Haube aus zwei übereinanderliegenden Folienschichten zu fertigen. Diese Folienschichten bestehen bevorzugt aus Polyethylen oder Polyvinylchlorid. Die Folie im Inneren der Haube weist bevorzugt keine vorgefertigten Löcher auf. Ferner trägt die äußere Folie das bevorzugt bereits vorgestanzte Lochraster.

Die Löcher des Lochrasters oder deren Vorzeichnung haben bevorzugt einen Durchmesser von 0.1 bis 0.75 mm. Die, gegebenenfalls nur vorgezeichneten, Löcher sind bevorzugt derart angeordnet, daß sie auf den imaginären Linien eines Gitternetzes liegen. Das Gitternetz besteht bevorzugt aus Quadraten. Alle, gegebenenfalls nur vorgezeichneten, Löcher besitzen entlang der Gitternetzlinien einen gleichmäßigen Abstand, der bevorzugt 0.5 bis 2 cm beträgt.

Die Haube hat bevorzugt die Gestalt eines Schiffchens oder eines Helms. Besonders bevorzugt ist die Helmform. Ganz besonders bevorzugt ist eine Haube, wie sie in den

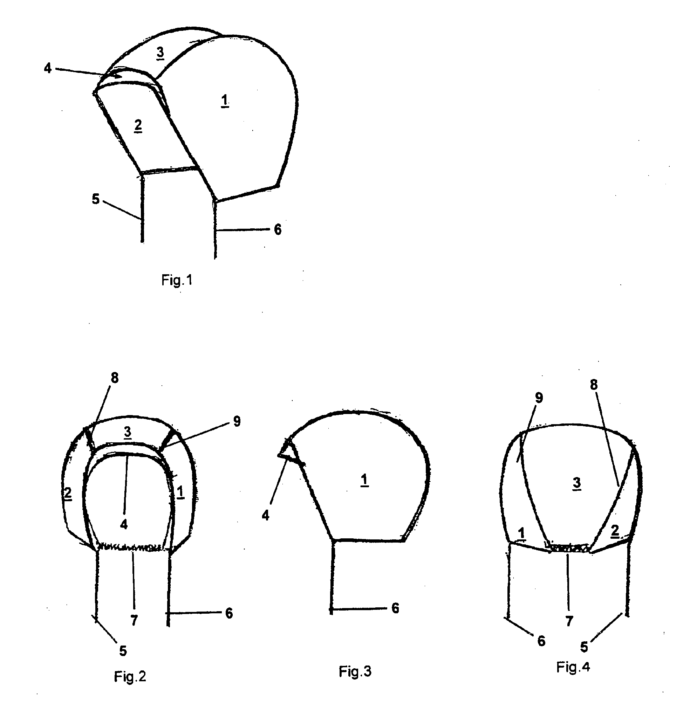

Fig. 1 bis 4 abgebildet wird. Diese Haube besteht primär aus zwei Seitenteilen (1) und (2) sowie einem Mittelteil (3) und einem Schirmteil (4). Diese Teile sind an deren Kontaktstellen bevorzugt durch eine geschweißte und/oder genähte Naht (8) und (9) miteinander verbunden. Die Nähte und die außenliegenden Ränder der Teile (1), (2), (3) und (4) sind mit einem Saum umsäumt. Der Saum besteht bevorzugt aus dem Material der außenliegenden Folie. Die Teile (1), (2) und (3) der Haube bestehen aus 2 übereinanderliegenden Folien. Die äußere Folie besitzt ein vorgestanztes Lochraster, die innere Folie weist keine Löcher auf. Beide übereinanderliegende Folien werden zum einen durch die Verbindungsnaht der Teile (1), (2) und (3), sowie zum anderen durch den Saum miteinander verbunden. Die Haube deckt, wenn sie über den Kopf gezogen wird, den Hinterkopf ab und besitzt vorne eine Öffnung für das Gesicht (siehe Fig. 2, der "Frontansicht"). Am oberen Teil des Gesichtsfeldes ist der Schirmteil (4) befestigt. Der Rand des Schirmteils und die äußere Kante der Teile (1), (2) und (3) werden wie zuvor beschrieben durch einen Saum eingefaßt. Der Saum wird am unteren Bereich des Gesichtsfeldes über den Rand der Haube hinaus verlängert und bildet rechts und links die Bänder (5) und (6), mit denen die Haube am Kopf durch Zusammenknoten der Bänder unter dem Kinn befestigt werden kann. Fig. 2 und Fig. 4 zeigen den Gummizug (7), welcher entlang der unteren Kante des Mittelteils (3) eingearbeitet ist. Durch den Gummizug wird ein straffer Sitz der Haube gewährleistet. Die Haube paßt sich mit Hilfe des Gummizuges an unterschiedliche Kopfgrößen an.

Die Einwirkzeit Z1 beträgt bevorzugt 5 bis 60 Minuten, besonders bevorzugt 20 bis 45 Minuten.

In einer weiteren Ausführungsform des Verfahrens werden die abgeteilten und mit dem Nuanciermittel behandelten Faserbündel in eine Folie, bevorzugt in Aluminiumfolie, eingewickelt und über die Dauer der Einwirkzeit Z1 in dieser Folie belassen. Diese Ausführungsform kann bevorzugt bei kürzeren Einwirkzeiten von bis zu 30 Minuten verwendet werden.

Die Nuanciermittel des erfindungsgemäßen Verfahrens enthalten Wasserstoffperoxid. Das Wasserstoffperoxid wird als Lösung oder in Form einer festen Anlagerungsverbindung von Wasserstoffperoxid an anorganische oder organische Verbindungen, wie beispielsweise Natriumperborat, Natriumpercarbonat, Magnesiumpercarbonat, Natriumpercarbamid, Polyvinylpyrrolidon·n H₂O₂ (n ist eine positive ganze Zahl größer 0), Harnstoffperoxid und Melaminperoxid, dem Nuanciermittel zugesetzt. In den Nuanciermitteln ist Wasserstoffperoxid bevorzugt in einer Menge von 0,5 bis 6,0 Gew.%, bezogen auf das Gewicht des Nuanciermittels, enthalten.

Die Nuanciermittel des erfindungsgemäßen Verfahrens enthalten bevorzugt zusätzliche Peroxoverbindungen. Darunter sind solche Peroxoverbindungen zu verstehen, die weder Wasserstoffperoxid selbst, noch Anlagerungsprodukte von Wasserstoffperoxid an andere Komponenten darstellen. Die Auswahl der in den erfindungsgemäßen Mitteln zusätzlich enthaltenen Peroxoverbindungen unterliegt prinzipiell keinen Beschränkungen; übliche, dem Fachmann bekannte Peroxoverbindungen sind beispielsweise Ammoniumperoxidisulfat, Kaliumperoxidisulfat, Natriumperoxidisulfat, Ammoniumpersulfat, Kaliumpersulfat, Natriumpersulfat, Kaliumperoxidiphosphat und Peroxide wie Magensium- und Bariumperoxid. Unter diesen Peroxoverbindungen, die auch in Kombination eingesetzt werden können, sind erfindungsgemäß die anorganischen Verbindungen bevorzugt. Besonders bevorzugt sind die Peroxidisulfate, insbesondere Ammoniumperoxidisulfat.

Die Peroxoverbindungen sind in den erfindungsgemäßen Nuanciermitteln bevorzugt in Mengen von 1 bis 40 Gew.-%, insbesondere in Mengen von 2 bis 35 Gew.-%, enthalten. Der pH-Wert der erfindungsgemäßen Mittel liegt bevorzugt in einem pH-Bereich von pH 2.5 bis 12.0, besonders bevorzugt von pH 8.5 bis 11.0.

Die Nuanciermittel des erfindungsgemäßen Verfahrens enthalten als bevorzugtes Alkalisierungsmittel mindestens eine Verbindung, ausgewählt aus Ammonium-, Alkalimetall- und Erdalkalimetallhydroxiden, -carbonaten, -hydrogencarbonaten, -hydroxycarbonaten und -carbamiden, sowie Alkaliphosphaten.

Ferner kann das Nuanciermittel zusätzlich mindestens eine SiO₂-Verbindung, die gegebenenfalls hydratisiert sein kann, enthalten. Es kann erfindungsgemäß bevorzugt sein, die gegebenenfalls hydratisierten SiO₂-Verbindungen in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.-% bis 5 Gew.-%, jeweils bezogen auf das gesamte Nuanciermittel, einzusetzen. Die Mengenangaben geben dabei jeweils den Gehalt der SiO₂-Verbindungen (ohne deren Wasseranteil) in den Mitteln wieder. Hinsichtlich der gegebenenfalls hydratisierten SiO₂-Verbindungen unterliegt die vorliegende Erfindung prinzipiell keinen Beschränkungen. Bevorzugt sind Kieselsäuren, deren Oligomeren und Polymeren sowie deren Salze. Bevorzugte Salze sind die Alkalisalze, insbesondere die Kalium und Natriumsalze. Die Natriumsalze sind ganz besonders bevorzugt.

Die gegebenenfalls hydratisierten SiO₂-Verbindungen können in verschiedenen Formen vorliegen. Erfindungsgemäß bevorzugt werden die SiO₂-Verbindungen in Form von Kieselgelen (Silicagel) oder besonders bevorzugt als Wasserglas eingesetzt. Diese SiO₂-Verbindungen können teilweise in wäßriger Lösung vorliegen.

Erfindungsgemäß ganz besonders bevorzugt sind Wassergläser, die aus einem Silikat der Formel (SiO₂)ₙ(Na₂O)ₘ(K₂O)ₚ gebildet werden, wobei n steht für eine positive rationale Zahl und m und p stehen unabhängig voneinander für eine positive rationale Zahl oder für 0, mit den Maßgaben, daß mindestens einer der Parameter m oder p von 0 verschieden ist und das Verhältnis zwischen n und der Summe aus m und p zwischen 1:4 und 4:1 liegt.

Neben den durch die Summenformel beschriebenen Komponenten können die Wassergläser in geringen Mengen noch weitere Zusatzstoffe, wie beispielsweise Phosphate oder Magnesiumsalze, enthalten.

Erfindungsgemäß besonders bevorzugte Wassergläser werden unter anderem von der Firma Henkel unter den Bezeichnungen Ferrosil^{®} 119, Natronwasserglas 40/42, Portil^{®} A, Portil^{®} AW, Portil^{®} N und Portil^{®} W und von der Firma Akzo unter der Bezeichnung Britesil^{®} C20 vertrieben.

Das Nuanciermittel besitzt eine bevorzugte Viskosität von 5000 bis 100000 mPa·s, besonders bevorzugt von 30000 bis 80000 mPa·s (Brookfield Rotationsviskosimeter, 25°C, Spindel #4, 20 rpm).

Die Viskosität wird durch das in dem Nuanciermittel enthaltene Verdickungsmittel eingestellt. Polymere können die Viskosität von wäßrigen und nicht-wäßrigen Phasen in kosmetischen Zubereitungen erhöhen. In wäßrigen Phasen beruht ihre die Viskosität erhöhende Funktion auf ihrer Löslichkeit in Wasser oder ihrer hydrophilen Natur. Sie werden sowohl in tensidischen als auch in emulsionsförmigen Systemen angewendet. Im folgenden werden einige Beispiele bevorzugter polymerer Verdicker angeführt, die in dem Nuanciermittel des erfindungsgemäßen Verfahrens enthalten sein können:
Acrylamides Copolymer, Acrylamide/Sodium Acrylate Copolymer, Acrylamide/Sodium Acryloyldimethyltaurate Copolymer, Acrylates/Acetoacetoxyethyl Methacrylate Copolymer, Acrylates/Beheneth-25 Methacrylate Copolymer, Acrylates/C10-30 Alkyl Acrylate Crosspolymer, Acrylates/Ceteth-20 Itaconate Copolymer, Acrylates/Ceteth-20 Methacrylate Copolymer, Acrylates/Laureth-25 Methacrylate Copolymer, Acrylates/Palmeth-25 Acrylate Copolymer, Acrylates/Palmeth-25 Itaconate Copolymer, Acrylates/Steareth-50 Acrylate Copolymer, Acrylates/Steareth-20 Itaconate Copolymer, Acrylates/Steareth-20 Methacrylate Copolymer, Acrylates/Stearyl Methacrylate Copolymer, Acrylates/Vinyl Isodecanoate Crosspolymer, Acrylic Acid/Acrylonitrogens Copolymer, Agar, Agarose, Alcaligenes Polysaccharides, Algin, Alginic Acid, Ammonium Acrylates/Acrylonitrogens Copolymer, Ammonium Acrylates Copolymer, Ammonium AcryloyldimethyltaurateNinyl Formamide Copolymer, Ammonium AcryloyldimethyltaurateNP Copolymer, Ammonium Alginate, Ammonium Polyacryloyldimethyl Taurate, Amylopectin, Ascorbyl Methylsilanol Pectinate, Astragalus Gummifer Gum, Attapulgite, Avena Sativa (Oat) Kernel Flour, Bentonite, Butoxy Chitosan, Caesalpinia Spinosa Gum, Calcium Alginate, Calcium Carboxymethyl Cellulose, Calcium Carrageenan, Calcium Potassium Carbomer, Calcium Starch Octenylsuccinate, C20-40 Alkyl Stearate, Carbomer, Carboxybutyl Chitosan, Carboxymethyl Chitin, Carboxymethyl Chitosan, Carboxymethyl Dextran, Carboxymethyl Hydroxyethylcellulose, Carboxymethyl Hydroxypropyl Guar, Cellulose Acetate Propionate Carboxylate, Cellulose Gum, Ceratonia Siliqua Gum, Cetyl Hydroxyethylcellulose, Cholesterol/HDI/Pullulan Copolymer, Cholesteryl Hexyl Dicarbamate Pullulan, Cyamopsis Tetragonoloba (Guar) Gum, Diglycol/CHDM/Isophthalates/SIP Copolymer, Dihydrogenated Tallow Benzylmonium Hectorite, Dimethicone Crosspolymer-2, Dimethicone Propyl PG-Betaine, DMAPA Acrylates/Acrylic Acid/Acrylonitrogens Copolymer, Ethylene/Sodium Acrylate Copolymer, Gelatin, Gellan Gum, Glyceryl Alginate, Glycine Soja (Soybean) Flour, Guar Hydroxypropyltrimonium Chloride, Hectorite, Hydrated Silica, Hydrogenated Potato Starch, Hydroxybutyl Methylcellulose, Hydroxyethyl Acrylate/Sodium Acryloyldimethyl Taurate Copolymer, Hydroxyethylcellulose, Hydroxyethyl Chitosan, Hydroxyethyl Ethylcellulose, Hydroxypropylcellulose, Hydroxypropyl Chitosan, Hydroxypropyl Ethylenediamine Carbomer, Hydroxypropyl Guar, Hydroxypropyl Methylcellulose, Hydroxypropyl Methylcellulose Stearoxy Ether, Hydroxypropyl Starch, Hydroxypropyl Starch Phosphate, Hydroxypropyl Xanthan Gum, Hydroxystearamide MEA, Isobutylene/Sodium Maleate Copolymer, Lithium Magnesium Silicate, Lithium Magnesium Sodium Silicate, Macrocystis Pyrifera (Kelp), Magnesium Alginate, Magnesium Aluminum Silicate, Magnesium Silicate, Magnesium Trisilicate, Methoxy PEG-22/Dodecyl Glycol Copolymer, Methylcellulose, Methyl Ethylcellulose, Methyl Hydroxyethylcellulose, Microcrystalline Cellulose, Montmorillonite, Moroccan Lava Clay, Natto Gum, Nonoxynyl Hydroxyethylcellulose, Octadecene/MA Copolymer, Pectin, PEG-800, PEG-Crosspolymer, PEG-150/Decyl Alcohol/SMDI Copolymer, PEG-175 Diisostearate, PEG-190 Distearate, PEG-15 Glyceryl Tristearate, PEG-140 Glyceryl Tristearate, PEG-240/HDI Copolymer Bis-Decyltetradeceth-20 Ether, PEG-100/IPDI Copolymer, PEG-180/Laureth-50/TMMG Copolymer, PEG-10/Lauryl Dimethicone Crosspolymer, PEG-15/Lauryl:Dimethicone Crosspolymer, PEG-2M, PEG-5M, PEG-7M, PEG-9M, PEG-14M, PEG-20M, PEG-23M, PEG-25M, PEG-45M, PEG-65M, PEG-90M, PEG-115M, PEG-160M, PEG-120 Methyl Glucose Trioleate, PEG-180/Octoxynol-40/TMMG Copolymer, PEG-150 Pentaerythrityl Tetrastearate, PEG-4 Rapeseedamide, PEG-150/Stearyl Alcohol/SMDI Copolymer, Polyacrylate-3, Polyacrylic Acid, Polycyclopentadiene, Polyether-1, Polyethylene/Isopropyl Maleate/MA Copolyol, Polymethacrylic Acid, Polyquaternium-52, Polyvinyl Alcohol, Potassium Alginate, Potassium Aluminum Polyacrylate, Potassium Carbomer, Potassium Carrageenan, Potassium Polyacrylate, Potato Starch Modified, PPG-14 Laureth-60 Hexyl Dicarbamate, PPG-14 Laureth-60 Isophoryl Dicarbamate, PPG-14 Palmeth-60 Hexyl Dicarbamate, Propylene Glycol Alginate, PVP/Decene Copolymer, PVP Montmorillonite, Rhizobian Gum, Ricinoleic Acid/Adipic Acid/AEEA Copolymer, Sclerotium Gum, Sodium Acrylate/Acryloyldimethyl Taurate Copolymer, Sodium Acrylates/Acrolein Copolymer, Sodium Acrylates/Acrylonitrogens Copolymer, Sodium Acrylates Copolymer, Sodium AcrylatesNinyl Isodecanoate Crosspolymer, Sodium Acrylate/Vinyl Alcohol Copolymer, Sodium Carbomer, Sodium Carboxymethyl Chitin, Sodium Carboxymethyl Dextran, Sodium Carboxymethyl Beta-Glucan, Sodium Carboxymethyl Starch, Sodium Carrageenan, Sodium Cellulose Sulfate, Sodium Cyclodextrin Sulfate, Sodium Hydroxypropyl Starch Phosphate, Sodium Isooctylene/MA Copolymer, Sodium Magnesium Fluorosilicate, Sodium Polyacrylate, Sodium Polyacrylate Starch, Sodium Polyacryloyldimethyl Taurate, Sodium Polymethacrylate, Sodium Polystyrene Sulfonate, Sodium Silicoaluminate, Sodium Starch Octenylsuccinate, Sodium Stearoxy PG-Hydroxyethylcellulose Sulfonate, Sodium Styrene/Acrylates Copolymer, Sodium Tauride Acrylates/Acrylic Acid/Acrylonitrogens Copolymer, Solanum Tuberosum (Potato) Starch, Starch/Acrylates/Acrylamide Copolymer, Starch Hydroxypropyltrimonium Chloride, Steareth-60 Cetyl Ether, Steareth-100/PEG-136/HDI Copolymer, Sterculia Urens Gum, Synthetic Fluorphlogopite, Tamarindus Indica Seed Gum, Tapioca Starch, TEA-Alginate, TEA-Carbomer, Triticum Vulgare (Wheat) Starch, Tromethamine Acrylates/Acrylonitrogens Copolymer, Tromethamine Magnesium Aluminum Silicate, Welan Gum, Xanthan Gum, Yeast Beta-Glucan, Yeast Polysaccharides, Zea Mays (Corn) Starch.

Das Färbemittel des erfindungsgemäßen Verfahrens ist ein oxidatives Färbemittel und enthält mindestens zwei Entwicklerkomponenten gemäß Anspruch 1. Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterozyklische Hydrazone, 4-Amino-pyrazolderivate sowie 2,4,5,6-Tetraaminopyrimidin und dessen Derivate eingesetzt.

Es ist erfindungsgemäß, dass das Färbemittel eine Kombination aus
(a) mindestens einem p-Phenylendiaminderivate der Formel (E1) 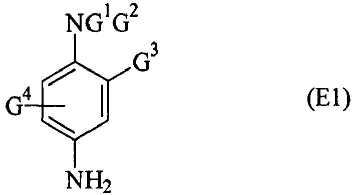 wobei gilt:
   - G¹ steht für ein Wasserstoffatom, einen C₁- bis C₄-Alkylrest, einen C₁- bis C₄-Mono-hydroxyalkylrest, einen C₂- bis C₄-Polyhydroxyalkylrest, einen (C₁- bis C₄)-Alkoxy-(C₁- bis C₄)-alkylrest, einen 4'-Aminophenylrest oder einen C₁- bis C₄-Alkylrest, der mit einer stickstoffhaltigen Gruppe, einem Phenyl- oder einem 4'-Aminophenylrest substituiert ist;
   - G² steht für ein Wasserstoffatom, einen C₁- bis C₄-Alkylrest, einen C₁- bis C₄-Mono-hydroxyalkylrest, einen C₂- bis C₄-Polyhydroxyalkylrest, einen (C₁- bis C₄)-Alkoxy-(C₁- bis C₄)-alkylrest oder einen C₁- bis C₄-Alkylrest, der mit einer stickstoffhaltigen Gruppe substituiert ist;
   - G³ steht für ein Wasserstoffatom, ein Halogenatom, wie ein Chlor-, Brom-, lod- oder Fluoratom, einen C₁- bis C₄-Alkylrest, einen C₁- bis C₄-Monohydroxyalkylrest, einen C₂- bis C₄-Polyhydroxyalkylrest, einen C₁- bis C₄-Hydroxyalkoxyrest, einen C₁- bis C₄-Acetylaminoalkoxyrest, einen C₁- bis C₄- Mesylaminoalkoxyrest oder einen C₁- bis C₄-Carbamoylaminoalkoxyrest;
   - G⁴ steht für ein Wasserstoffatom, ein Halogenatom oder einen C₁- bis C₄-Alkylrest oder
   - wenn G³ und G⁴ in ortho-Stellung zueinander stehen, können sie gemeinsam eine verbrückende α,ω-Alkylendioxogruppe, wie beispielsweise eine Ethylendioxygruppe, bilden,
(b) mindestens einem p-Aminophenolderivat der Formel (E3) 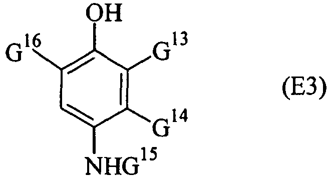 wobei gilt:
   - G¹³ steht für ein Wasserstoffatom, ein Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest, einen Hydroxy-(C₁- bis C₄)-alkylaminorest, einen C₁- bis C₄-Hydroxyalkoxyrest, einen C₁-bis C₄-Hydroxyalkyl-(C₁-bis C₄)-aminoalkylrest oder einen (Di-C₁- bis C₄-Alkylamino)-(C₁- bis C₄)-alkylrest, und
   - G¹⁴ steht für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen (C₁-bis C₄)-Alkoxy-(C₁- bis C₄)-alkylrest, einen C₁- bis C₄-Aminoalkylrest oder einen C₁-bis C₄-Cyanoalkylrest,
   - G¹⁵ steht für Wasserstoff, einen C₁- bis C₄-Alkylrest, einen C₁- bis C₄-Monohydroxyalkylrest, einen C₂- bis C₄-Polyhydroxyalkylrest, einen Phenylrest oder einen Benzylrest, und
   - G¹⁶ steht für Wasserstoff oder ein Halogenatom,
(c) mindestens einem Pyridinderivat, ausgewählt aus 2,6-Dihydroxypyridin, 2-Amino-3-hydroxypyridin, 2-Amino-5-chlor-3-hydroxypyridin, 3-Amino-2-methylamino-6-methoxy-pyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin, als Kuppler, sowie
(d) mindestens einer Verbindung, ausgewählt aus m-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol, als Kuppler enthält.

Beispiele für die als Substituenten in den erfindungsgemäßen Verbindungen genannten C₁- bis C₄-Alkylreste sind die Gruppen Methyl, Ethyl, Propyl, Isopropyl und Butyl. Ethyl und Methyl sind bevorzugte Alkylreste. Erfindungsgemäß bevorzugte C₁- bis C₄-Alkoxy-reste sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte C₂- bis C₄-Polyhydroxyalkylgruppe ist die 1,2-Dihydroxyethylgruppe. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (E1) sind insbesondere die Aminogruppen, C₁- bis C₄-Monoalkylaminogruppen, C₁- bis C₄-Dialkylaminogruppen, C₁- bis C₄-Trialkylammoniumgruppen, C₁- bis C₄-Monohydroxyalkylaminogruppen, Imidazolinium und Ammonium.

Besonders bevorzugte p-Phenylendiamine der Formel (E1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)amino-2-methylanilin, 4-N,N-Bis-(β-hydroxyethyl)amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin und 5,8-Diaminobenzo-1,4-dioxan sowie ihren physiologisch verträglichen Salzen. Erfindungsgemäß ganz besonders bevorzugte p-Phenylendiaminderivate der Formel (E1) sind p-Phenylendiamin, p-Toluylendiamin, 2-(β-Hydroxyethyl)-p-phenylendiamin, 2-(α,β-Dihydroxyethyl)-p-phenylendiamin und N,N-Bis-(β-hydroxyethyl)-p-phenylendiamin. Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Es kann erfindungsgemäß weiterhin bevorzugt sein, als zusätzliche Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbemitteln gemäß der Erfindung bevorzugt verwendet werden können, kann man insbesondere die Verbindungen nennen, die der folgenden Formel (E2) entsprechen, sowie ihre physiologisch verträglichen Salze: 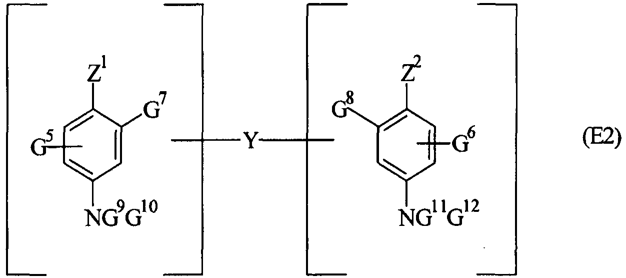 wobei:
- Z¹ und Z² stehen unabhängig voneinander für einen Hydroxyl- oder NH₂-Rest, der gegebenenfalls durch einen C₁- bis C₄-Alkylrest, durch einen C₁- bis C₄-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder C₁- bis C₈-Alkoxyreste substituiert sein kann, oder eine direkte Bindung,
- G⁵ und G⁶ stehen unabhängig voneinander für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen C₁- bis C₄-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen C₁- bis C₄-Alkylrest,
mit den Maßgaben, dass
- die Verbindungen der Formel (E2) nur eine Verbrückung Y pro Molekül enthalten und
- die Verbindungen der Formel (E2) mindestens eine Aminogruppe enthalten, die mindestens ein Wasserstoffatom trägt.

Die in Formel (E2) verwendeten Substituenten sind erfindungsgemäß analog zu den obigen Ausführungen definiert.

Bevorzugte zweikernige Entwicklerkomponenten der Formel (E2) sind insbesondere: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Diethyl-N,N'-bis-(4'-amino-3'-methylphenyl)-ethylendiamin, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten der Formel (E2) sind N,N'-Bis-(β-hydroxyethyl)-N,N'-bis-(4'-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan und 1,10-Bis-(2',5'-diaminophenyl)-1,4,7,10-tetraoxadecan oder eines ihrer physiologisch verträglichen Salze.

Ferner kann eine zusätzliche Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann eine zusätzliche Entwicklerkomponente ausgewählt sein aus heterozyklischen Entwicklerkomponenten, wie beispielsweise den Pyridin-, Pyrimidin-, Pyrazol-, Pyrazol-Pyrimidin-Derivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4'-Methoxyphenyl)amino-3-amino-pyridin, 2,3-Diamino-6-methoxy-pyridin, 2-(ß-Methoxyethyl)amino-3-amino-6-methoxy-pyridin und 3,4-Diamino-pyridin.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin. Bevorzugte Pyrazol-Derivate sind insbesondere die Verbindungen, die in den Patenten DE 3 843 892, DE 4 133 957 und Patentanmeldungen WO 94/08969, WO 94/08970, EP-740 931 und DE 195 43 988 beschrieben werden, wie 4,5-Diamino-1-methylpyrazol, 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropyl-pyrazol, 4-Amino-5-(2-aminoethyl)amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4-(β-hydroxyethyl)amino-1-methylpyrazol.

Bevorzugte PyrazoloPyrimidin-Derivate sind insbesondere die Derivate des Pyrazolo[1,5-a]pyrimidin der folgenden Formel (E4) und dessen tautomeren Formen, sofern ein tautomeres Gleichgewicht besteht: 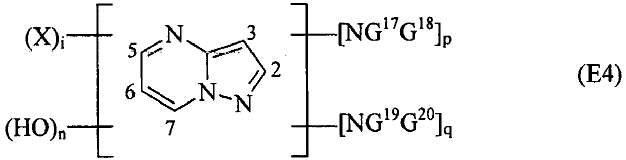 wobei:
- G¹⁷, G¹⁸, G¹⁹ und G²⁰ unabhängig voneinander stehen für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest einen (C₁- bis C₄)-Alkoxy-(C₁- bis C₄)-alkylrest, einen C₁- bis C₄-Aminoalkylrest, der gegebenenfalls durch ein Acetyl-Ureid- oder einen Sulfonyl-Rest geschützt sein kann, einen (C₁- bis C₄)-Alkylamino-(C₁- bis C₄)-alkylrest, einen Di-[(C₁- bis C₄)-alkyl]-(C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄)-[Hydroxyalkyl]-(C₁- bis C₄)-aminoalkylrest,
- die X-Reste stehen unabhängig voneinander für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest, einen C₁- bis C₄-Aminoalkylrest, einen (C₁- bis C₄)-Alkylamino-(C₁- bis C₄)-alkylrest, einen Di-[(C₁- bis C₄)alkyl]- (C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁-bis C₄-hydroxyalkyl)aminoalkylrest, einen Aminorest, einen C₁- bis C₄-Alkyl- oder Di-(C₁- bis C₄-hydroxyalkyl)aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
mit der Maßgabe, dass
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG¹⁷G¹⁸ (oder NG¹⁹G²⁰) und die Gruppe OH belegen die Positionen (2,3); (5,6); (6,7); (3,5) oder (3,7).

Die in Formel (E4) verwendeten Substituenten sind erfindungsgemäß analog zu den obigen Ausführungen definiert.

Wenn das Pyrazolo[1,5-a]pyrimidin der obenstehenden Formel (E4) eine Hydroxygruppe an einer der Positionen 2, 5 oder 7 des Ringsystems enthält, besteht ein tautomeres Gleichgewicht, das zum Beispiel im folgenden Schema dargestellt wird:

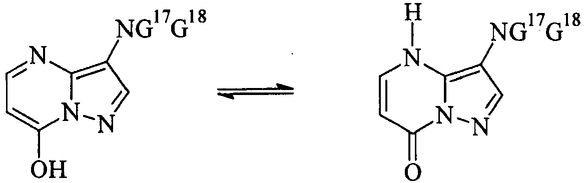

Unter den Pyrazolo[1,5-a]pyrimidinen der obenstehenden Formel (E4) kann man insbesondere nennen:
- Pyrazolo[1,5-a]pyrimidin-3,7-diamin;
- 2,5-Dimethyl-pyrazolo[1,5-a]pyrimidin-3,7-diamin;
- Pyrazolo[1,5-a]pyrimidin-3,5-diamin;
- 2,7-Dimethyl-pyrazolo[1,5-a]pyrimidin-3,5-diamin;
- 3-Aminopyrazolo[1,5-a]pyrimidin-7-ol;
- 3-Aminopyrazolo[1,5-a]pyrimidin-5-ol;
- 2-(3-Aminopyrazolo[1,5-a]pyrimidin-7-ylamino)-ethanol;
- 2-(7-Aminopyrazolo[1,5-a]pyrimidin-3-ylamino)-ethanol;
- 2-[(3-Aminopyrazolo[1,5-a]pyrimidin-7-yl)-(2-hydroxy-ethyl)amino]-ethanol;
- 2-[(7-Aminopyrazolo[1,5-a]pyrimidin-3-yl)-(2-hydroxy-ethyl)amino]-ethanol;
- 5,6-Dimethylpyrazolo[1,5-a]pyrimidin-3,7-diamin;
- 2,6-Dimethylpyrazolo[1,5-a]pyrimidin-3,7-diamin;
- 3-Amino-7-dimethylamino-2,5-dimethylpyrazolo[1,5-a]pyrimidin;
sowie ihre physiologisch verträglichen Salze und ihre tautomeren Formen, wenn ein tautomeres Gleichgewicht vorhanden ist.

Die Pyrazolo[1,5-a]pyrimidine der oben stehenden Formel (E4) können, wie in der Literatur beschrieben, durch Zyklisierung ausgehend von einem Aminopyrazol oder von Hydrazin hergestellt werden.

Als Vorstufen naturanaloger Farbstoffe werden bevorzugt solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. Diese Indole bzw. Indoline können als Entwicklerkomponente in Oxidationshaarfarben Verwendung finden.

Besonders gut als Vorstufen naturanaloger Haarfarbstoffe geeignet sind Derivate des 5,6-Dihydroxyindolins der Formel (la), 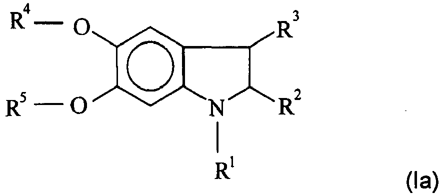 in der unabhängig voneinander
- R¹ steht für Wasserstoff, eine C₁-C₄-Alkylgruppe oder eine C₁-C₄-Hydroxy-alkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (Ib), 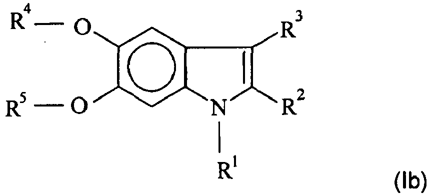 in der unabhängig voneinander
- R¹ steht für Wasserstoff, eine C₁-C₄-Alkylgruppe oder eine C₁-C₄-Hydroxyalkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
- sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den im Rahmen eines erfindungsgemäß bevorzugten Verfahrens eingesetzten Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.-%, vorzugsweise 0,2-5 Gew.-% enthalten.

In einer weiteren Ausführungsform kann es erfindungsgemäß bevorzugt sein, das Indolin- oder Indolderivat in Haarfärbemitteln in Kombination mit mindestens einer Aminosäure oder einem Oligopeptid einzusetzen. Die Aminosäure ist vorteilhafterweise eine α-Aminosäure; ganz besonders bevorzugte α-Aminosäuren sind Arginin, Ornithin, Lysin, Serin und Histidin, insbesondere Arginin.

Die Färbemittel des erfindungsgemäßen Verfahrens enthalten mindestens zwei Kupplerkomponenten gemäß Anspruch 1. Als Kupplerkomponenten werden in der Regel m-Phenylendiaminderivate, Naphthole, Resorcin und Resorcinderivate, Pyrazolone und m-Aminophenolderivate sowie heterozyklische Verbindungen verwendet.

Erfindungsgemäß bevorzugte zusätzliche Kupplerkomponenten sind
- o-Aminophenol und dessen Derivate,
- m-Diaminobenzol und dessen Derivate wie beispielsweise 2,4-Diaminophenoxyethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Amino-benzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylen-dioxybenzol, 1-Amino-3,4-methylendioxybenzol und 1-(2'-Hydroxyethyl)amino-3,4-methylendioxybenzol.

Erfindungsgemäß besonders bevorzugte Kupplerkomponenten sind 1-Naphthol, 1,5-Dihydroxynaphthalin, 2,7-Dihydroxynaphthalin, 1,7-Dihydroxynaphthalin, 5-Amino-2-methylphenol, m-Aminophenol, Resorcin, m-Phenylendiamin, 1-Phenyl-3-methyl-pyrazol-5-on, 2,4-Dichlor-3-aminophenol, 1,3-Bis-(2,4-diaminophenoxy)-propan, 2-Chlor-resorcin, 4-Chlor-resorcin, 2-Chlor-6-methyl-3-aminophenol, 3-Amino-2-methylamino-6-methoxypyridin, 2-Amino-3-hydroxypyridin, 2,6-Dihydroxy-3,4-dimethylpyridin, 2-Methylresorcin, 5-Methylresorcin, 2-Methyl-4-chlor-5-aminophenol und physiologisch verträgliche Salze der vorgenannten Verbindungen.

Nach Durchführung des erfindungsgemäßen Verfahrens ergeben sich Farbpaare, die aus der Färbung des Schritts C und der vorangegangenen Aufhellung im Schritt A des erfindungsgemäßen Verfahrens gebildet werden. Als Grundfärbung wird erfindungsgemäß die erhaltene Färbung der Faser auf nicht zuvor aufgehellter Faser definiert. Die Färbung auf der aufgehellten Faser ergibt eine aufgehellte Färbung in dem Grundfarbton der Grundfärbung. Die Bildung solcher erfindungsgemäßen Farbpaare läßt sich farbmetrisch bestimmen.

Es ist besonders bevorzugt die Färbemittel derart zu formulieren, daß sich nach der Durchführung des erfindungsgemäßen Verfahrens eines der 10 Farbpaare für die Ausgangsfärbung und das oxidativ nuancierte Haar gemäß Tabelle 1 ergibt:

**Tabelle 1**

| Nr. | Farbe der Grundfärbung | Farbe der aufgehellten Färbung |
|---|---|---|
| 1 | Mittelblond | Blond |
| 2 | Dunkelblond | Hellblond |
| 3 | Rot | Rot-orange |
| 4 | Rot | Hellrot |
| 5 | Kupferrot | Hellkupferblond |
| 6 | Violett | Dunkelrosa |
| 7 | Goldbraun | Goldblond |
| 8 | Mittelbraun | braunes Blond |
| 9 | Dunkelbraun | Hellbraun |
| 10 | Schwarzbraun | Braun |

Es wurde überraschenderweise gefunden, daß sich folgende Kombinationen von Oxidationsfarbstoffvorprodukten zur Darstellung von Farbpaaren mit blonder Ausgangsfärbung (Farbpaare 1 und 2 gemäß Tabelle 1) besonders gut eignen:
- mindestens ein p-Phenylendiaminderivat gemäß Formel (E1)
- mindestens ein p-Aminophenolderivat gemäß Formel (E3)
- mindestens ein Pyridinderivat als Kuppler
- mindestens eine Verbindung ausgewählt aus m-Aminophenol oder seiner Derivate als Kuppler.

Zur Bildung der Farbpaare werden in den oben genannten Kombinationen als bevorzugte Entwickler bzw. Kuppler die zuvor erwähnten bevorzugten Oxidationsfarbstoffvorprodukte verwendet.

Die Färbemittel des erfindungsgemäßen Verfahrens enthalten sowohl die Entwicklerkomponenten als auch die Kupplerkomponenten bevorzugt in einer Menge von 0,005 bis 10 Gew.-%, vorzugsweise von 0,1 bis 5 Gew.-%, jeweils bezogen auf das gesamte Oxidationsfärbemittel.

Dabei werden Entwicklerkomponenten und Kupplerkomponenten im allgemeinen in etwa molaren Mengen zueinander eingesetzt. Wenn sich auch der molare Einsatz als zweckmäßig erwiesen hat, so ist ein gewisser Überschuß einzelner Oxidationsfarbstoffvorprodukte nicht nachteilig, so daß Entwicklerkomponenten und Kupplerkomponenten in einem Mol-Verhältnis von 1:0,5 bis 1:3, insbesondere 1:1 bis 1:2, enthalten sein können.

Zur Nuancierung können die Färbemittel des erfindungsgemäßen Verfahrens einen oder mehrere direktziehende Farbstoffe enthalten. Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)amino-2-nitrobenzol, 3-Nitro-4-(β-hydroxyethyl)aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol. Ferner können die Färbemittel einen kationischen direktziehenden Farbstoff enthalten. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterozyklus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

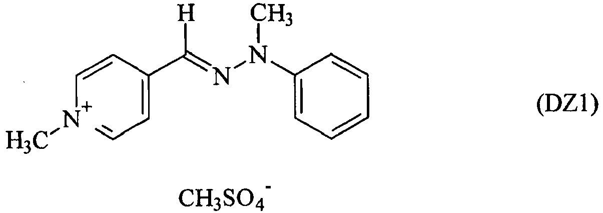

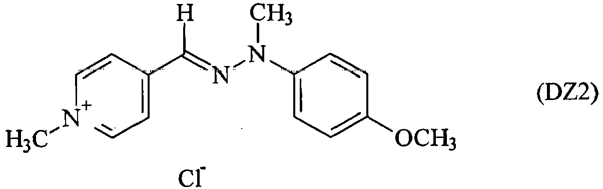

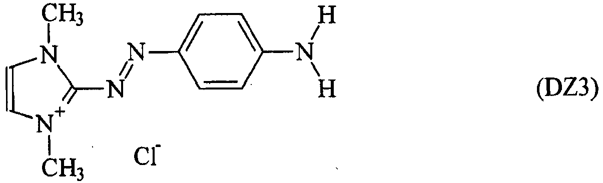

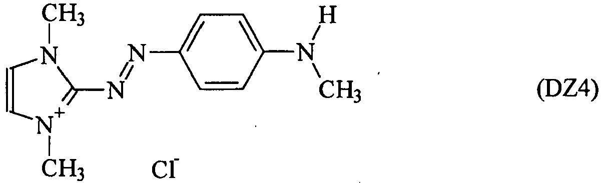

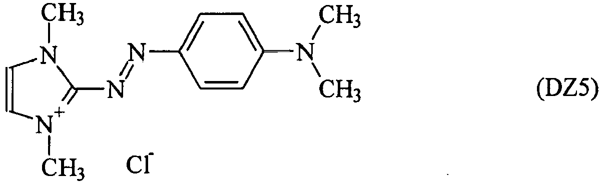

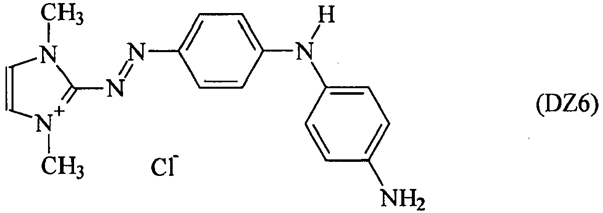

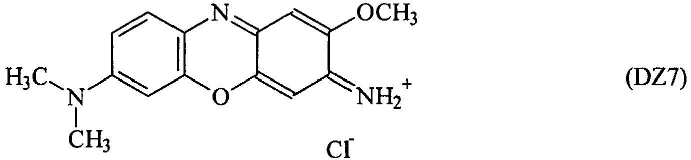

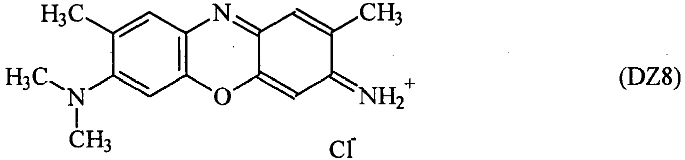

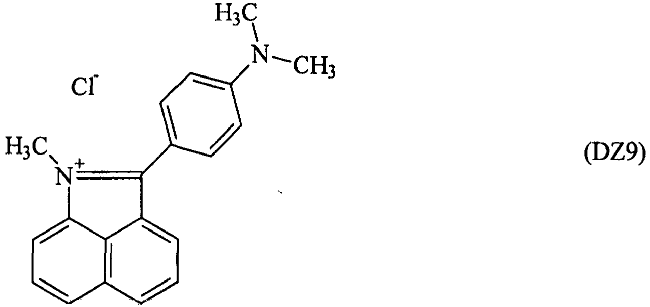

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor^{®} vertrieben werden, sind erfindungsgemäß ebenfalls ganz besonders bevorzugte kationische direktziehende Farbstoffe.

Bevorzugte Färbemittel können die direktziehenden Farbstoffe in einer Menge von 0,1 bis 5 Gew.-%, bezogen auf das gesamte Färbemittel, enthalten.

Weiterhin können die Färbemittel des erfindungsgemäßen Verfahrens auch in der Natur vorkommende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Es ist jedoch erfindungsgemäß bevorzugt, daß die in dem erfindungsgemäßen Verfahren verwendeten Färbemittel keine direktziehenden Farbstoffe enthalten.

Bezüglich der in dem Färbemittel des erfindungsgemäßen Verfahrens einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Eine oxidative Färbung der Fasern kann in Gegenwart von Oxidationsfarbstoffvorprodukten grundsätzlich mit Luftsauerstoff erfolgen. Bevorzugt wird jedoch ein chemisches Oxidationsmittel eingesetzt, besonders dann, wenn neben der Färbung ein Aufhelleffekt der natürlichen Pigmente des menschlichen Haars gewünscht ist. Dieser Aufhelleffekt kann unabhängig von der Färbemethode gewünscht sein. Die Gegenwart von Oxidationsfarbstoffvorprodukten ist demnach keine zwingende Voraussetzung für einen Einsatz von Oxidationsmitteln in den Färbemitteln des erfindungsgemäßen Verfahrens. Als Oxidationsmittel kommt insbesondere Wasserstoffperoxid oder dessen Anlagerungsprodukte an Harnstoff, Melamin sowie Natriumborat in Frage. Das eigentliche Färbemittel wird bei getrennter Lagerung der Farbstoffvorprodukte und des Oxidationsmittels unmittelbar vor der Anwendung durch Mischen hergestellt.

Es ist erfindungsgemäß bevorzugt, die Färbemittel des erfindungsgemäßen Verfahrens frei von Peroxoverbindungen zu formulieren. Unter Peroxoverbindungen werden die Verbindungen definiert, wie sie im Rahmen der bevorzugten Ausführungsformen des Nuanciermittels beschrieben werden.

Erfindungsgemäß kann aber das Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z.B. Metallionen, Iodide, Chinone oder bestimmte Enzyme.

Geeignete Metallionen sind beispielsweise Zn²⁺, Cu²⁺, Fe²⁺, Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflusst werden.

Geeignete Enzyme sind z.B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder *in situ* geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z.B.
- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Bei einer Anwendung von Oxidationsmitteln wird das eigentliche Färbemittel zweckmäßigerweise unmittelbar vor der Anwendung durch Mischung der Zubereitung des Oxidationsmittels mit der Zubereitung, enthaltend die Verbindungen der Formel I und gegebenenfalls Farbstoffvorprodukte, hergestellt. Das dabei entstehende gebrauchsfertige Haarfärbepräparat sollte bevorzugt einen pH-Wert im Bereich von 6 bis 12 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Insbesondere bei schwer färbbarem Haar kann ein erfindungsgemäßes Mittel gegebenenfalls mit zusätzlichen Farbstoffvorprodukten aber auch ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

Die Mittel des erfindungsgemäßen Verfahrens enthalten die erfindungsgemäßen Inhaltsstoffe bevorzugt in einem geeigneten wäßrigen, alkoholischen oder wäßrig-alkoholischen kosmetischen Träger. Solche Träger sind beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Es ist aber auch denkbar, die Inhaltsstoffe in eine pulverförmige oder auch tablettenförmige Formulierung zu integrieren.

Unter wäßrig-alkoholischen Lösungen sind im Sinne der vorliegenden Erfindung wäßrige Lösungen enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Die Färbemittel sowie die Nuanciermittel des erfindungsgemäßen Verfahrens können weiterhin alle für solche Zubereitungen bekannten Wirk-, Zusatz- und Hilfsstoffe enthalten.

In vielen Fällen enthalten die Färbemittel und/oder die Nuanciermittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel R¹O-(Z)_{X}. Diese Verbindungen sind durch die folgenden Parameter gekennzeichnet.

Der Alkylrest R¹ enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R¹ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R¹
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, dass eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat. Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin. Erfindungsgemäß werden als kationische Tenside insbesondere solche vom Typ der quartären Ammoniumverbindungen, der Esterquats und der Amidoamine eingesetzt. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxy-ethyl)dimethylammoniumchlorid, sowie Dehyquart^{®} F-75 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyldimethylamin dar.

Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Ein Beispiel für ein als kationisches Tensid einsetzbares quaternäres Zuckerderivat stellt das Handelsprodukt Glucquat^{®}100 dar, gemäß INCI-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Ferner können die Färbemittel und die Nuanciermittel des erfindungsgemäßen Verfahrens weitere Wirk-, Hilfs- und Zusatzstoffe, wie beispielsweise
- nichtionische Polymere wie beispielsweise Vinylpyrrolidon/Vinylacrylat-Copolymere, Polyvinylpyrrolidon und VinylpyrrolidonNinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazolinium-methochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, VinylpyrrolidonNinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butyl-acrylamid-Terpolymere,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,.
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wasserstoffperoxid und andere Oxidationsmittel,
- Treibmittel wie Propan-Butan-Gemische, N₂O, Dimethylether, CO₂ und Luft,
- Antioxidantien,
enthalten.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Die Anwendungstemperaturen des Färbemittels können in einem Bereich zwischen 15 und 40 °C liegen. Nach einer Einwirkungszeit Z2 von bevorzugt 2 bis 60 Minuten, besonders bevorzugt von 5 bis 45 Minuten, wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Es ist erfindungswesentlich, direkt im Anschluß an Schritt A bzw. Schritt B den Färbeschritt C des erfindungsgemäßen Verfahrens durchzuführen. Der Zeitraum, der als direkter Anschluß im Sinne der Erfindung definiert ist, beträgt maximal 90 Minuten. Das bevorzugte Zeitintervall zwischen der vollendeten Durchführung des Schritts A des erfindungsgemäßen Verfahrens und dem Beginn der Durchführung des Schritts C sollte nicht länger als 60 Minuten, besonders bevorzugt nicht länger als 45 Minuten, ganz besonders bevorzugt nicht länger als 20 Minuten lang sein.

Ein zweiter Gegenstand der vorliegenden Anmeldung ist ein Kit, enthaltend
- gegebenenfalls einen Applikator,
- einen Container C1, enthaltend ein Färbemittel,
- einen Container C2a, enthaltend eine wasserstoffperoxidhaltige Zusammensetzung und
- einen Container C2b, enthaltend eine Zusammensetzung, enthaltend mindestens ein Verdickungsmittel und mindestens ein Alkalisierungsmittel,
wobei das Färbemittel als farbgebende Komponente
mindestens zwei Oxidationsfarbstoffvorprodukte enthält, dadurch gekennzeichnet, dass das Färbemittel eine Kombination aus
(a) mindestens einem p-Phenylendiaminderivat gemäß Formel (E1), 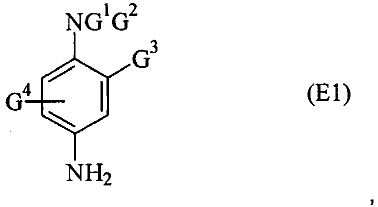 wobei
   - G¹ steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen 4'-Aminophenylrest' oder einen C₁- bis C₄-Alkylrest, der mit einer stickstoffhaltigen Gruppe, einem Phenyl- oder einem 4'-Aminophenylrest substituiert ist;
   - G² steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest oder einen C₁- bis C₄-Alkylrest, der mit einer stickstoffhaltigen Gruppe substituiert ist;
   - G³ steht für ein Wasserstoffatom, ein Halogenatom, wie ein Chlor-, Brom-, lod- oder Fluoratom, einen C₁- bis C₄-Alkylrest, einen C₁- bis C₄-Monohydroxyalkyl-rest, einen C₂- bis C₄-Polyhydroxyalkylrest, einen C₁- bis C₄-Hydroxyalkoxyrest, einen C₁- bis C₄-Acetylaminoalkoxyrest, einen C₁- bis C₄- Mesylaminoalkoxyrest oder einen C₁- bis C₄-Carbamoylaminoalkoxyrest;
   - G⁴ steht für ein Wasserstoffatom, ein Halogenatom oder einen C₁- bis C₄-Alkyl-rest oder
   - wenn G³ und G⁴ in ortho-Stellung zueinander stehen, können sie gemeinsam eine verbrückende α,ω-Alkylendioxogruppe bilden,
(b) mindestens einem p-Aminophenolderivat gemäß Formel (E3), 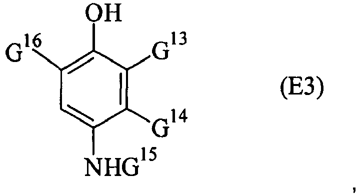 wobei:
   - G¹³ steht für ein Wasserstoffatom, ein Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest, einen Hydroxy-(C₁- bis C₄)-alkylaminorest, einen C₁- bis C₄-Hydroxyalkoxyrest, einen C₁- bis C₄-Hydroxyalkyl-(C₁-bis C₄)-aminoalkylrest oder einen (Di-C₁- bis C₄-Alkylamino)-(C₁- bis C₄)-alkylrest, und
   - G¹⁴ steht für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest oder einen C₁- bis C₄-Cyanoalkylrest,
   - G¹⁵ steht für Wasserstoff, einen C₁- bis C₄-Alkylrest, einen C₁- bis C₄-Monohydroxyalkylrest, einen C₂- bis C₄-Polyhydroxyalkylrest, einen Phenylrest oder einen Benzylrest, und
   - G¹⁶ steht für Wasserstoff oder ein Halogenatom,
(c) mindestens einem Pyridinderivat, ausgewählt aus 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin, als Kuppler, sowie
(d) mindestens einer Verbindung, ausgewählt aus m-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol, als Kuppler enthält.

Es ist bevorzugt, daß das aus Mischung des Inhalts aus Container C2a und C2b resultierende Nuanciermittel eine Viskosität von 5000 bis 100000 mPa·s (Brookfield Rotationsviskosimeter, 25°C, Spindel #4, 20 rpm) besitzt.

Das Färbemittel und das Nuanciermittel als Mischung von C2a und C2b besitzen die bevorzugten Merkmale, wie sie im ersten Gegenstand der Erfindung beschrieben wurden. Als Applikatoren dienen die im ersten Gegenstand der Erfindung genannten Applikatoren. Des Weiteren ist es erfindungsgemäß bevorzugt, dem Kit zusätzlich eine Haube mit, gegebenenfalls nur vorgezeichnetem, Lochraster, und eine Häkelnadel beizufügen. Die Haube besitzt die bevorzugten Merkmale, wie sie bereits im ersten Gegenstand der Erfindung beschrieben wurden.

Das Färbemittel liegt bevorzugt in zwei Containern C1a und C1b in dem Kit vor. Wenn es sich um ein oxidatives Färbemittel handelt, befindet sich in Container C1a die sogenannte Färbecreme, welche die Farbstoffvorprodukte enthält, und im Container 1b wird eine Wasserstoffperoxidhaltige Zusammensetzung aufbewahrt.

Wenn es sich um ein Färbemittel der Oxofärbung handelt, können die Verbindungen der Komponente A in Container C1a und die Verbindungen der Komponente B in Container C1 b getrennt gelagert werden.

Ein dritter Gegenstand der Erfindung ist die Verwendung des Kits gemäß zweitem Gegenstand der Erfindung in einem Verfahren des ersten Gegenstands der Erfindung.

### Beispiele

Zur Herstellung der nachfolgenden Rezepturbeispiele wurden folgende Rohstoffe verwendet:

| | |
|---|---|
| Hydrenol^{®} D | C₁₆-C₁₈ Fettalkohol (INCI-Bezeichnung: Cetearyl Alcohol) (Cognis) |
| Texapon^{®} NSO | Natriumlaurylsulfat (INCI: Sodium Lauryl Sulfate) (Cognis) |
| Lorol^{®} techn. | C₁₂-C₁₈-Fettalkohol (INCI-Bezeichnung: Coconut Alcohol) (Cognis) |
| Lorol^{®} C 16 | Cetylalkohol (Cognis) |
| Dehyton^{®} K | N,N-Dimethyl-N-(C₈-C₁₈-kokosamidopropyl)-ammoniumacetobetain (ca. 30 % Aktivsubstanz; INCI-Bezeichnung: Aqua (Water), Cocamidopropyl Betaine) (Cognis) |
| Aculyn^{®} 33 | (INCI-Bezeichnung: Acrylates Copolymer) (Rohm & Haas) |
| Turpinal^{®} SL | 1-Hydroxyethan-1,1-diphosphonsäure (INCI-Bezeichnung: Etidronic Acid, Aqua (Water)) (Solutia) |
| Natronwasserglas 40/42 | Natriumsilikat (Cognis) |
| Idranal^{®} III | Ethylendiamintetraessigsäure Dinatrium Salz 2 H₂O (INCI-Bezeichnung: Disodium EDTA) (Hersteller: Riedel De Haen) |
| Britesil^{®} C20 | Natriumsilikat (INCI-Bezeichnung: Sodium Silikate) (The PQ Corporation) |
| Eumulgin^{®} B2 | Cetylstearylalkohol mit ca. 20 EO-Einheiten (INCI-Bezeichnung: Ceteareth-20) |
| Lanette^{®} E | Cetylstearylalkoholsulfat-Natrium-Salz (INCI-Bezeichnung: Sodium Cetearyl Sulfate) (Cognis) |
| Ceasit^{®} I | C16-18 Fettsäure Calcium Salz (INCI-Bezeichnung:: Calcium Stearate) (Bärlocher) |
| Aerosil^{®} 200 | Siliciumdioxid (INCI-Bezeichnung: Silica) (Degussa) |

### 1. Rezepturen des Oxidationshaarfärbemittels

Es wird eine Färbecreme gemäß Tabelle 2 hergestellt:

**Tabelle 2: Färbecreme**

| Rohstoff | Menge in Gew.% |
|---|---|
| Hydrenol D | 8.0 |
| Lorol techn. | 2.0 |
| Texapon NSO | 16.0 |
| Dehyton K | 10.0 |
| Ascorbinsäure | 0.4 |
| Natriumsulfit | 0.5 |
| Ammoniumchlorid | 0.5 |
| Turpinal SL | 0.2 |
| Natronwasserglas 40/42 | 0.5 |
| m-Aminophenol | 0.02 |
| Resorcin | 0.13 |
| p-Toluylendiamin | 0.36 |
| 2,7-Dihydroxynaphthalin | 0.10 |
| 2-Methylresorcin | 0.03 |
| 3-Amino-2-methylamino-6-methoxypyridin | 0.015 |
| 2-Amino-3-hydroxypyridin | 0.001 |
| 3-Methyl-4-aminophenol | 0.03 |
| Parfum | 0.2 |
| Ammoniak (25%ige wässrige Lösung) | 0.6 |
| Wasser | ad 100 |

### 2. Rezepturen des Nuanciermittels

Es werden folgende Zusammensetzungen gemäß Tabellen 3 und 4 hergestellt:

**Tabelle 3: Nuancierpulver**

| Rohstoff | Menge in Gew.-% |
|---|---|
| Ammoniumperoxidisulfat | 21,5 |
| Natriumphosphat | 4,0 |
| Aerosil 200 | 3,0 |
| Kaliumpersulfat | 33,0 |
| Britesil C20 | 22,0 |
| Natriumstearat | 8,0 |
| Ceasit | 4,0 |
| Magnesiumoxid | 2,0 |
| Magnesiumhydroxidcarbonat | 1,0 |
| Lanette E | 1,0 |
| Idranal III | 0,5 |

**Tabelle 4: Wasserstoffperoxidlösung**

| Rohstoff | Menge in Gew.-% |
|---|---|
| Lorol C16 | 3,6 |
| Eumulgin | 0,9 |
| Texapon NSO | 2,25 |
| Ammoniak (25%) | 0,65 |
| Dipicolinsäure | 0,1 |
| Natriumpyrophosphat | 0,03 |
| Turpinal SL | 1,5 |
| H₂O₂ | 6 |
| Wasser | ad 100 |

### 3. Beurteilung des Farbergebnisses gemäß erfindungsgemäßem Verfahren

### 3.1 Durchführung des Schritts A des erfindungsgemäßen Verfahrens

25.0 g des Nuancierpulvers gemäß Tabelle 3 wird mit 50 mL der Zusammensetzung gemäß Tabelle 4 unter Erhalt des Nuanciermittels vermischt.

Auf ein abgeteiltes Faserbündel, bestehend aus ca. einem Drittel der Haarfasern einer Humanhaarsträhne (1 g, Kerling Naturweiß), werden 1 g des Nuanciermittels mit Hilfe einer Mascarabürste gleichmäßig über den gesamten Bereich des Faserbündels aufgetragen, für eine Dauer von 10 Minuten bei 32°C auf dem Haar belassen und anschließend abgespült. Die Haarsträhne wurde mit dem Handtuch getrocknet und behielt eine Restfeuchtigkeit.

### 3.2 Durchführung des Schritts C des erfindungsgemäßen Verfahrens

Kurz vor der Anwendung wurde eine Färbecreme gemäß Tabelle 2 mit der Oxidationsmittelzubereitung gemäß Tabelle 5 im Gewichtsverhältnis 1:1 vermischt.

**Tabelle 5: Oxidationsmittelzubereitung**

| Rohstoff | Menge in Gew.% |
|---|---|
| Wasserstoffperoxid | 6.00 |
| Aculyn^{®} 33 | 3.40 |
| Texapon^{®} NSO | 2.00 |
| Turpinal SL | 1.50 |
| Natriumpyrophosphat | 0.03 |
| Dipicolinsäure | 0.10 |
| Ammoniak (25%ige wäßrige Lösung) | 0.62 |
| Wasser | ad 100 |

2 g der zuvor hergestellten Mischung wurden auf die gesamte gemäß Punkt 3.1 behandelte Haarsträhne aufgetragen, für eine Dauer von 30 Minuten bei 32°C auf dem Haar belassen und anschließend abgespült. Die Haarsträhne wurde getrocknet und das Farbergebnis des nuancierten Haars beurteilt. Das Haar erhielt eine blonde Färbung mit hellblonden Farbreflexen in dem nuancierten Bereich.

### 4. Anwendung einer Haube mit Lochraster

Das trockene Kopfhaar einer Testperson mit hellblondem Ausgangshaar wird mit einer Haube mit Lochraster abgedeckt. Durch die Löcher der Haube werden, gleichmäßig über die gesamte Oberfläche der Haube verteilt, vereinzelt Haarsträhnen mit Hilfe einer Häkelnadel hindurchgezogen. Die hindurchgezogenen Haarsträhnen werden gekämmt. Das Nuanciermittel wird aus 25.0 g des Nuancierpulvers gemäß Tabelle 3 und 50 mL der Zusammensetzung gemäß Tabelle 4 durch Mischen hergestellt und mit einem Pinsel auf die Haarsträhnen aufgetragen. Nach einer Einwirkzeit von 30 Minuten wird das Nuanciermittel abgespült und die Haube vom Kopf entfernt und das Haar mit einem Handtuch getrocknet, so daß eine Restfeuchtigkeit zurückblieb (erfindungsgemäßer Schritt A).

Es wurde ein Haarfärbemittel zubereitet, in dem die Färbecreme aus Tabelle 2 mit der Oxidationsmittelzubereitung gemäß Tabelle 5 in einem Gewichtsverhältnis von 1:1 gemischt wurde.

Auf das gesamte Kopfhaar der Testperson wurde dieses Haarfärbemittel aufgetragen und nach einer Einwirkzeit von 30 Minuten wieder abgespült (Schritt C des erfindungsgemäßen Verfahrens).

Es wird ein im Grundton blondes Haar mit hellblonden Strähnchen sowohl im Deckhaar als auch in darunterliegenden Haarbereichen erhalten. Die Strähnchen sind gleichmäßig vom Haaransatz bis in die Spitzen nuanciert und bilden einen scharfen Kontrast zum nicht nuancierten Haar.

## Patentansprüche

1. Verfahren zum Färben keratinhaltiger Fasern, Insbesondere menschlicher Haare, in welchem
A auf einen Teil der Fasern ein Nuanciermittel, enthaltend in einem kosmetischen Träger mindestens ein Verdickungsmittel, Wasserstoffperoxid und mindestens ein Alkalisierungsmittel aufgetragen und nach einer Einwirkzeit Z1 wieder abgespült wird,
B die Fasern im Anschluß gegebenenfalls getrocknet werden und anschließend
C auf die gesamten Fasern ein Färbemittel, enthaltend farbgebende Komponenten, aufgetragen und nach einer Einwirkzeit Z2 wieder abgespült wird,
wobei das Färbemittel als farbgebende Komponente mindestens zwei Oxidationsfarbstoffvorprodukte enthält,
**dadurch gekennzeichnet, dass** das Färbemittel eine Kombination aus
(a) mindestens einem p-Phenylendiaminderivat gemäß Formel (E1), 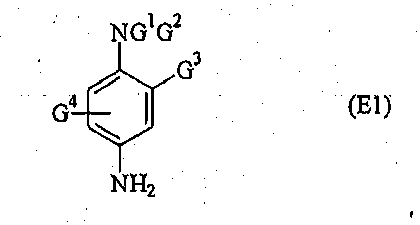 wobei
- G¹ steht für ein Wasserstoffatom, einen C₁- bis C_{A}-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen 4'-Aminophenylrest oder einen C₁- bis C₄-Alkylrest, der mit einer stickstoffhaltigen Gruppe, einem Phenyl- oder einem 4'-Aminophenylrest substituiert ist;
- G² steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest oder einen C₁- bis C₄-Alkylrest, der mit einer stickstoffhaltigen Gruppe substituiert ist;
- G³ steht für ein Wasserstoffatom, ein Halogenatom, wie ein Chlor-, Brom-, lod- oder Fluoratom, einen C₁- bis C₄-Alkylrest, einen C₁- bis C₄-Monohydroxyalkylrest, einen C₂- bis C₄-Polyhydroxyalkylrest, einen C₁- bis C₄-Hydroxyalkoxyrest, einen C₁- bis C₄-Acetylaminoalkoxyrest, einen C₁- bis C₄-Mesylaminoalkoxyrest oder einen C₁- bis C₄-Carbamoylaminoalkoxyrest;
- G⁴ steht für ein Wasserstoffatom, ein Halogenatom oder einen C₁- bis C₄-Alkylrest oder
- wenn G³ und G⁴ in ortho-Stellung zueinander stehen, können sie gemeinsam eine verbrückende α,ω-Alkylendioxogruppe bilden,
(b) mindestens einem p-Aminophenolderivat gemäß Formel (E3), 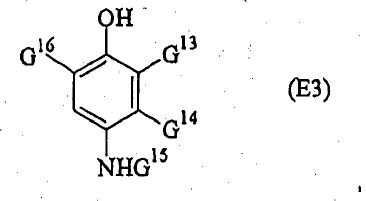 wobei:
- G¹³ steht für ein Wasserstoffatom, ein Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C4)-alkylrest, einen C₁- bis C₄-Aminoalkylrest, einen Hydroxy-(C₁- bis C₄)-alkylaminorest, einen C₁- bis C₄-Hydroxyalkoxyrest, einen C₁- bis C₄-Hydroxyalkyl-(C₁-bis C₄)-aminoalkylrest oder einen (Di-C₁- bis C₄-Alkylamino)-(C₁- bis C₄)-alkylrest, und
- G¹⁴ steht für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest oder einen C₁- bis C₄-Cyanoalkylrest,
- G¹⁶ steht für Wasserstoff, einen C₁- bis C₄-Alkylrest, einen C₁- bis C₄-Monohydroxyalkylrest, einen C₂- bis C₄-Polyhydroxyalkylrest, einen Phenylrest oder einen Benzylrest, und
- G¹⁶ steht für Wasserstoff oder ein Halogenatom,
(c) mindestens einem Pyridinderivat, ausgewählt aus 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin, als Kuppler, sowie
(d) mindestens einer Verbindung, ausgewählt aus m-Aminophenol, 5-Amino-2-methylphenol, N-Cyclopentyl-3-aminophenol, 3-Amino-2-chlor-6-methylphenol, 2-Hydroxy-4-aminophenoxyethanol, 2,8-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol, als Kuppler enthält.

2. Verfahren nach Anspruch 1, **dadurch gekennzeichnet, daß** das Nuanciermittel zusätzlich mindestens eine Peroxoverbindung enthält.

3. Verfahren nach einem der Ansprüche 1 oder 2, **dadurch gekennzeichnet, daß** das Nuanciermittel mittels eines Applikators, ausgewählt aus der Gruppe, die gebildet wird aus Bürste, Pinsel und Applicette, auf einen Teil der zuvor gefärbten keratinhaltigen Fasern aufgetragen wird.

4. Verfahren nach einem der Ansprüche 1 bis 3, **dadurch gekennzeichnet daß** das Nuanciermittel eine Viskosität von 5000 bis 100000 mPa·s (Brookfield Rotationsviskosimeter, 25°C, Spindel #4, 20 rpm) besitzt.

5. Verfahren nach einem der Ansprüche 1 bis 4, **dadurch gekennzeichnet, daß** vor dem Schritt A die keratinhaltigen Fasern mit einer Haube, enthaltend ein, gegebenenfalls nur vorgezeichnetes, Lochraster, abgedeckt werden, durch die Löcher ausgewählte Faserbündel hindurchgezogen werden, dann Schritt A erfolgt und danach die Haube wieder entfernt wird.

6. Verfahren nach einem der Ansprüche 1 bis 5, **dadurch gekennzeichnet, daß** zwischen dem abgeschlossenen Schritt A und dem Beginn des Schritts C maximal 60 Minuten liegen.

7. Verfahren nach einem der Ansprüche 1 bis 6, **dadurch gekennzeichnet, daß** das Färbemittel frei ist von direktziehenden Farbstoffen.

8. Kit enthaltend
• gegebenenfalls einen Applikator, einen Container C1, enthaltend ein Färbemittel,
• einen Container C2a, enthaltend eine wasserstoffperoxidhaltige Zusammensetzung und
• einen Container C2b, enthaltend eine Zusammensetzung, enthaltend mindestens ein Verdickungsmittel und mindestens ein Alkalisierungsmittel, wobei das Färbemittel als farbgebende Komponente mindestens zwei Oxidationsfarbstoffvorprodukte enthält, **dadurch gekennzeichnet, dass** das Färbemittel eine Kombination aus
(a) mindestens einem p-Phenylendiaminderivat gemäß Formel (E1), 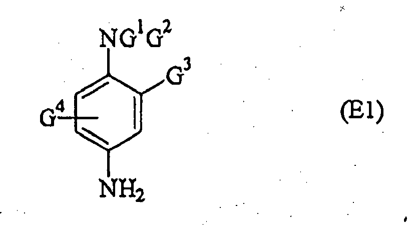 wobei
- G¹ steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest einen 4'-Aminophenylrest oder einen C₁- bis C₄-Alkylrest, der mit einer stickstoffhaltigen Gruppe, einem Phenyl- oder einem 4'-Aminophenylrest substituiert ist;
- G² steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest oder einen C₁- bis C₄-Alkylrest, der mit einer stickstoffhaltigen Gruppe substituiert ist;
- G³ steht für ein Wasserstoffatom, ein Halogenatom, wie ein Chlor-, Brom-, lod- oder Fluoratom, einen C₁- bis C₄-Alkylrest, einen C₁- bis C₄-Monohydroxyalkylrest, einen C₂- bis C₄-Polyhydroxyalkylrest, einen C₁- bis C₄-Hydroxyalkoxyrest, einen C₁- bis C₄-Acetylaminoalkoxyrest, einen C₁- bis C₄- Mesylaminoalkoxyrest oder einen C₁- bis C₄-Carbamoylaminoalkoxyrest;
- G⁴ steht für ein Wasserstoffatom, ein Halogenatom oder einen C₁- bis C₄-Alkylrest oder
- wenn G³ und G⁴ in ortho-Stellung zueinander stehen, können sie gemeinsam eine verbrückende α,ω-Alkylendioxogruppe bilden,
(b) mindestens einem p-Aminophenolderivat gemäß Formel (E3), 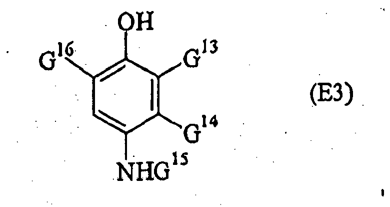 wobei:
- G¹³ steht für ein Wasserstoffatom, ein Halogenatom, einen C₁- bis C₄-Alkylrest, einen C₁- bis C₄-Monohydroxyalkylrest, einen C₂- bis C₄-Polyhydroxyalkylrest, einen (C₁ bis C₄)-Alkoxy-(C₁- bis C₄)-alkylrest, einen C₁- bis C₄-Aminoalkylrest, einen Hydroxy-(C₁- bis C₄)-alkylaminorest, einen C₁- bis C₄-Hydroxyalkoxyrest, einen C₁- bis C₄-Hydroxyalkyl-(C₁-bis C₄)-aminoalkylrest oder einen (Di-C₁- bis C₄-Alkylamino)-(C₁- bis C₄)-alkylrest, und
- G¹⁴ steht für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest oder einen C₁- bis C₄-Cyanoalkylrest,
- G¹⁵ steht für Wasserstoff, einen C₁- bis C₄-Alkylrest, einen C₁- bis C₄-Monohydroxyalkylrest, einen C₂- bis C₄-Polyhydroxyalkylrest, einen Phenylrest oder einen Benzylrest, und
- G¹⁶ steht für Wasserstoff oder ein Halogenatom,
(c) mindestens einem Pyridinderivat, ausgewählt aus 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5,Diamino-2,6-dimethoxypyridin, als Kuppler, sowie
(d) mindestens einer Verbindung, ausgewählt aus m-Aminophenol, 5-Amino2-methylphenol, N-Cyclopentyl-3-aminophenol, 3-Amino-2-chlor-6-methylphenol, 2-Hydroxy-4-aminophonoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol, als Kuppler enthält.

9. Kit nach Anspruch 8, **dadurch gekennzeichnet, daß** aus Mischung des Inhalts aus Container C2a und C2b das resultierende Nuanciermittel eine Viskosität von 5000 bis 100000 mPa·s (Brookfield Rotationsviskosimeter, 25°C, Spindel #4, 20 rpm) besitzt.

10. Kit nach Anspruch 8 oder 9, **dadurch gekennzeichnet, daß** die Oxidationsfarbstoffvorprodukte in einem Container C1a und eine wasserstoffperoxidhaltige Zusammensetzung in Container C1b getrennt konfektioniert sind.

11. Verwendung des Kits nach einem der Ansprüche 8 bis 10 in einem Verfahren zum Färben keratinhaltiger Fasern gemäß einem der Ansprüche 1 bis 7.

## Claims

1. A method for colouring keratin-containing fibres, in particular human hair, in which
A a shading agent containing in a cosmetic carrier at least one thickener, hydrogen peroxide and at least one alkalising agent is applied onto some of the fibres and rinsed off again after a period of exposure Z1,
B the fibres are then optionally dried and thereafter
C a colouring agent containing colour-imparting components is applied onto all of the fibres and rinsed off again after a period of exposure Z2,
wherein the colouring agent contains as colour-imparting component at least two oxidation dye precursors,
**characterised in that** the colouring agent contains a combination of
(a) at least one p-phenylenediamine derivative according to formula (E1), 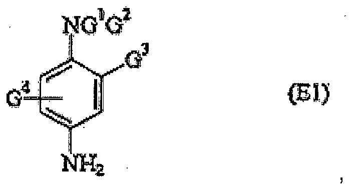 wherein
- G¹ denotes a hydrogen atom, a C₁ to C₄ alkyl residue, a C₁ to C₄ monohydroxyalkyl residue, a C₂ to C₄ polyhydroxyalkyl residue, a (C₁ to C₄)-alkoxy-(C₁ to C₄)-alkyl residue, a 4'-aminophenyl residue or a C₁ to C₄ alkyl residue, which is substituted with a nitrogenous group, a phenyl or a 4'-aminophenyl residue;
- G² denotes a hydrogen atom, a C₁ to C₄ alkyl residue, a C₁ to C₄ monohydroxyalkyl residue, a C₂ to C₄ polyhydroxyalkyl residue, a (C₁ to C₄)-alkoxy-(C₁ to C₄)-alkyl residue or a C₁ to C₄ alkyl residue, which is substituted with a nitrogenous group;
- G³ denotes a hydrogen atom, a halogen atom, such as a chlorine, bromine, iodine or fluorine atom, a C₁ to C₄ alkyl residue, a C₁ to C₄ monohydroxyalkyl residue, a C₂ to C₄ polyhydroxyalkyl residue, a C₁ to C₄ hydroxyalkoxy residue, a C₁ to C₄ acetylaminoalkoxy residue, a C₁ to C₄ mesylaminoalkoxy residue or a C₁ to C₄ carbamoylaminoalkoxy residue;
- G⁴ denotes a hydrogen atom, a halogen atom or a C₁ to C₄ alkyl residue or
- if G³ and G⁴ are in ortho position relative to one another, they may together form a bridging α,ω-alkylenedioxo group,
(b) at least one p-aminophenol derivative according to formula (E3), 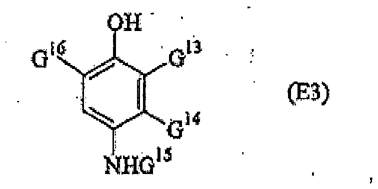 wherein
- G¹³ denotes a hydrogen atom, a halogen atom, a C₁ to C₄ alkyl residue, a C₁ to C₄ monohydroxyalkyl residue, a C₂ to C₄ polyhydroxyalkyl residue, a (C₁ to C₄)-alkoxy-(C₁ to C₄)-alkyl residue, a C₁ to C₄ aminoalkyl residue, a hydroxy-(C₁ to C₄)-alkylamino residue, a C₁ to C₄ hydroxyalkoxy residue, a C₁ to C₄-hydroxyalkyl-(C₁ to C₄)-aminoalkyl residue or a (di-C₁ to C₄-alkylamino)-(C₁ to C₄)-alkyl residue, and
- G¹⁴ denotes a hydrogen or halogen atom, a C₁ to C₄ alkyl residue, a C₁ to C₄ monohydroxyalkyl residue, a C₂ to C₄ polyhydroxyalkyl residue, a (C₁ to C₄)-alkoxy-(C₁ to C₄)-alkyl residue, a C₁ to C₄ aminoalkyl residue, or a C₁ to C₄ cyanoalkyl residue,
- G¹⁵ denotes hydrogen, a C₁- to C₄ alkyl residue, a C₁ to C₄ monohydroxyalkyl residue, a C₂ to C₄ polyhydroxyalkyl residue, a phenyl residue or a benzyl residue, and
- G¹⁶ denotes hydrogen or a halogen atom,
(c) at least one pyridine derivative, selected from 2,6-dihydroxypyridine, 2-amino-3-hydroxypyridine, 2-amino-5-chloro-3-hydroxypyridine, 3-amino-2-methylamino-6-methoxypyridine, 2,6-dihydroxy-3,4-dimethylpyridine, 2,6-dihydroxy-4-methylpyridine, 2,6-diaminopyridine, 2,3-diamino-6-methoxypyridine and 3,5-diamino-2,6-dimethoxypyridine, as coupler, together with
(d) at least one compound, selected from m-aminophenol, 5-amino-2-methylphenol, N-cyclopentyl-3-aminophenol, 3-amino-2-chloro-6-methylphenol, 2-hydroxy-4-aminophenoxyethanol, 2,6-dimethyl-3-aminophenol, I, 3-trifluoroacetylamino-2-chloro-6-methylphenol, 5-amino-4-chloro-2-methylphenol, 5-amino-4-methoxy-2-methylphenol, 5-(2'-hydroxyethyl)amino-2-methylphenol, 3-(diethylamino)phenol, N-cyclopentyl-3-aminophenol, 1,3-dihydroxy-5-(methylamino)benzene, 3-ethylamino-4-methylphenol and 2,4-dichloro-3-aminophenol, as coupler.

2. A method according to claim 1, **characterised in that** the shading agent additionally contains at least one peroxo compound.

3. A method according to either one of claims 1 or 2, **characterised in that** the shading agent is applied onto some of the previously coloured keratin-containing fibres by means of an applicator selected from the group formed by a brush, paintbrush and applicette.

4. A method according to any one of claims 1 to 3, **characterised in that** the shading agent has a viscosity of 5000 to 100000 mPa·s (Brookfield rotational viscometer, 25°C, spindle no. 4, 20 rpm).

5. A method according to any one of claims 1 to 4, **characterised in that**, prior to step A, the keratin-containing fibres are covered with a cap containing an optionally only predrawn hole pattern, selected bundles of fibres are pulled through the holes, then step A takes place and thereafter the cap is taken back off.

6. A method according to any one of claims 1 to 5, **characterised in that** at most 60 minutes elapse between the completion of step A and the start of step C.

7. A method according to any one of claims 1 to 6, **characterised in that** the colouring agent contains no substantive dyes.

8. A kit containing
• optionally an applicator,
• a container C1 containing a colouring agent,
• a container C2a containing a hydrogen peroxide-containing composition and
• a container C2b containing a composition containing at least one thickener and at least one alkalising agent,
wherein the colouring agent contains as colour-imparting component at least two oxidation dye precursors, **characterised in that** the colouring agent contains a combination of
(a) at least one p-phenylenediamine derivative according to formula (E1), 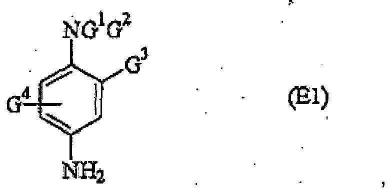 wherein
- G¹ denotes a hydrogen atom, a C₁ to C₄ alkyl residue, a C₁ to C₄ monohydroxyalkyl residue, a C₂ to C₄ polyhydroxyalkyl residue, a (C₁ to C₄)-alkoxy-(C₁ to C₄)-alkyl residue, a 4'-aminophenyl residue or a C₁ to C₄ alkyl residue, which is substituted with a nitrogenous group, a phenyl or a 4'-aminophenyl residue;
- G² denotes a hydrogen atom, a C₁ to C₄ alkyl residue, a C₁ to C₄ monohydroxyalkyl residue, a C₂ to C₄ polyhydroxyalkyl residue, a (C₁ to C₄)-alkoxy-(C₁ to C₄)-alkyl residue or a C₁ to C₄ alkyl residue, which is substituted with a nitrogenous group;
- G³ denotes a hydrogen atom, a halogen atom, such as a chlorine, bromine, iodine or fluorine atom, a C₁ to C₄ alkyl residue, a C₁ to C₄ monohydroxyalkyl residue, a C₂ to C₄ polyhydroxyalkyl residue, a C₁ to C₄ hydroxyalkoxy residue, a C₁ to C₄ acetylaminoalkoxy residue, a C₁ to C₄ mesylaminoalkoxy residue or a C₁ to C₄ carbamoylaminoalkoxy residue;
- G⁴ denotes a hydrogen atom, a halogen atom or a C₁ to C₄ alkyl residue or
- if G³ and G⁴ are in ortho position relative to one another, they may together form a bridging α,ω-alkylenedioxo group,
(b) at least one p-aminophenol derivative according to formula (E3), 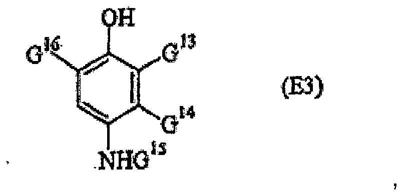 wherein
- G¹³ denotes a hydrogen atom, a halogen atom, a C₁ to C₄ alkyl residue, a C₁ to C₄ monohydroxyalkyl residue, a C₂ to C₄ polyhydroxyalkyl residue, a (C₁ to C₄)-alkoxy-(C₁ to C₄)-alkyl residue, a C₁ to C₄ aminoalkyl residue, a hydroxy-(C₁ to C₄)-alkylamino residue, a C₁ to C₄ hydroxyalkoxy residue, a C₁ to C₄-hydroxyalkyl-(C₁ to C₄)-aminoalkyl residue or a (di-C₁ to C₄-alkylamino)-(C₁ to C₄)-alkyl residue, and
- G¹⁴ denotes a hydrogen or halogen atom, a C₁ to C₄ alkyl residue, a C₁ to C₄ monohydroxyalkyl residue, a C₂ to C₄ polyhydroxyalkyl residue, a (C₁ to C₄)-alkoxy-(C₁ to C₄)-alkyl residue, a C₁ to C₄ aminoalkyl residue, or a C₁ to C₄ cyanoalkyl residue,
- G¹⁵ denotes hydrogen, a C₁ to C₄ alkyl residue, a C₁ to C₄ monohydroxyalkyl residue, a C₂ to C₄ polyhydroxyalkyl residue, a phenyl residue or a benzyl residue, and
- G¹⁶ denotes hydrogen or a halogen atom,
(c) at least one pyridine derivative, selected from 2,6-dihydroxypyridine, 2-amino-3-hydroxypyridine, 2-amino-5-chloro-3-hydroxypyridine, 3-amino-2-methylamino-6-methoxypyridine, 2,6-dihydroxy-3,4-dimethylpyridine, 2,6-dihydroxy-4-methylpyridine, 2,6-diaminopyridine, 2,3-diamino-6-methoxypyridine and 3,5-diamino-2,6-dimethoxypyridine, as coupler, together with
(d) at least one compound, selected from m-aminophenol, 5-amino-2-methylphenol, N-cyclopentyl-3-aminophenol, 3-amino-2-chloro-6-methylphenol, 2-hydroxy-4-aminophenoxyethanol, 2,6-dimethyl-3-aminophenol, 3-trifluoroacetylamino-2-chloro-6-methylphenol, 5-amino-4-chloro-2-methylphenol, 5-amino-4-methoxy-2-methylphenol, 5-(2'-hydroxyethyl)amino-2-methylphenol, 3-(diethylamino)phenol, N-cyclopentyl-3-aminophenol, 1,3-dihydroxy-5-(methylamino)benzene, 3-ethylamino-4-methylphenol and 2,4-dichloro-3-aminophenol, as coupler.

9. A kit according to claim 8, **characterised in that** the shading agent resulting from mixing the contents of containers C2a and C2b has a viscosity of 5000 to 100000 mPa·s (Brookfield rotational viscometer, 25°C, spindle no. 4, 20 rpm).

10. A kit according to claim 8 or claim 9, **characterised in that** the oxidation dye precursors are separately packaged in a container C1a and a hydrogen peroxide-containing composition in container C1b.

11. Use of the kit according to any one of claims 8 to 10 in a method for dyeing keratin-containing fibres according to any one of claims 1 to 7.

## Revendications

1. Procédé pour la teinture de fibres kératiniques, en particulier de cheveux humains, dans lequel
A un agent formant une nuance, contenant, dans un support cosmétique, au moins un épaississant, du peroxyde d'hydrogène et au moins un agent d'alcalinisation, est appliqué sur une partie des fibres, puis il est à nouveau éliminé par rinçage après un temps d'action Z1,
B les fibres sont ensuite le cas échéant séchées, puis
C un agent de teinture, contenant des composants colorants, est appliqué sur l'ensemble des fibres et à nouveau éliminé par rinçage après un temps d'action Z2,
l'agent de teinture contenant, comme composant colorant, au moins deux précurseurs de colorant par oxydation,
**caractérisé en ce que** l'agent de teinture contient une combinaison
(a) d'au moins un dérivé de p-phénylènediamine selon la formule (E1), 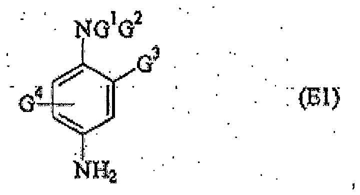 où
- G¹ représente un atome d'hydrogène, un résidu C₁-C₄-alkyle, un résidu C₁-C₄-monohydroxyalkyle, un résidu C₂-C₄-polyhydroxyalkyle, un résidu (C₁-C₄)-alcoxy-(C₁-C₄)-alkyle, un résidu 4'-aminophényle ou un résidu C₁-C₄-alkyle, qui est substitué par un groupe contenant azote, un résidu phényle ou un résidu 4'-aminophényle ;
- G² représente un atome d'hydrogène, un résidu C₁-C₄-alkyle, un résidu C₁-C₄-monohydroxyalkyle, un résidu C₂-C₄-polyhydroxyalkyle, un résidu (C₁-C₄)-alcoxy-(C₁-C₄)-alkyle ou un résidu C₁-C₄-alkyle, qui est substitué par un groupe contenant azote ;
- G³ représente un atome d'hydrogène, un atome d'halogène, tel qu'un atome de chlore, de brome, d'iode ou de fluor, un résidu C₁-C₄-alkyle, un résidu C₁-C₄-monohydroxyalkyle, un résidu C₂-C₄-polyhydroxyalkyle, un résidu C₁-C₄-hydroxyalcoxy, un résidu C₁-C₄-acétylaminoalcoxy, un résidu C₁-C₄-mésylaminoalcoxy ou un résidu C₁-C₄-carbamoylaminoalcoxy ;
- G⁴ représente un atome d'hydrogène, un atome d'halogène ou un résidu C₁-C₄-alkyle ou
- lorsque G³ et G⁴ sont en position ortho l'un par rapport à l'autre, ils peuvent former ensemble un groupe α,ω-alkylènedioxo formant un pont,
(b) d'au moins un dérivé de p-aminophénol selon la formule (E3), 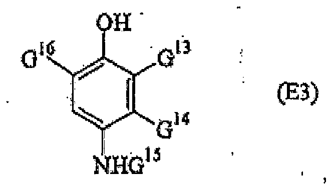 où
- G¹³ représente un atome d'hydrogène, un atome d'halogène, un résidu C₁-C₄-alkyle, un résidu C₁-C₄-monohydroxyalkyle, un résidu C₂-C₄-polyhydroxyalkyle, un résidu (C₁-C₄)-alcoxy-(C₁-C₄)-alkyle, un résidu C₁-C₄-aminoalkyle, un résidu hydroxy-(C₁-C₄)-alkylamino, un résidu C₁-C₄-hydroxyalcoxy, un résidu C₁-C₄-hydroxyalkyl-(C₁-C₄)-aminoalkyle ou un résidu (di-C₁-C₄-alkylamino)-(C₁₋C₄)-alkyle, et
- G¹⁴ représente un atome d'hydrogène ou d'halogène, un résidu C₁-C₄-alkyle, un résidu C₁-C₄-monohydroxyalkyle, un résidu C₂-C₄-polyhydroxyalkyle, un résidu (C₁-C₄)-alcoxy-(C₁-C₄)-alkyle, un résidu C₁-C₄-aminoalkyle ou un résidu C₁-C₄-cyanoalkyle,
- G¹⁵ représente hydrogène, un résidu C₁-C₄-alkyle, un résidu C₁-C₄-monohydroxyalkyle, un résidu C₂-C₄-polyhydroxyalkyle, un résidu phényle ou un résidu benzyle, et
- G¹⁶ représente hydrogène ou un atome d'halogène,
(c) d'au moins un dérivé de pyridine, choisi parmi la 2,6-dihydroxypyridine, la 2-amino-3-hydroxypyridine, la 2-amino-5-chloro-3-hydroxypyridine, la 3-amino-2-méthylamino-6-méthoxypyridine, la 2,6-dihydroxy-3,4-diméthylpyridine, la 2,6-dihydroxy-4-méthylpyridine, la 2,6-diaminopyridine, la 2,3-diamino-6-méthoxypyridine et la 3,5-diamino-2,6-diméthoxypyridine, comme agent de couplage, ainsi que
(d) d'au moins un composé choisi parmi le m-aminophénol, le 5-amino-2-méthylphénol, le N-cyclopentyl-3-aminophénol, le 3-amino-2-chloro-6-méthylphénol, le 2-hydroxy-4-aminophénoxyéthanol, le 2,6-diméthyl-3-aminophénol, le 3-trifluoroacétylamino-2-chloro-6-méthylphénol, le 5-amino-4-chloro-2-méthylphénol, le 5-amino-4-méthoxy-2-méthylphénol, le 5-(2'-hydroxyéthyl)amino-2-méthylphénol, le 3-(diéthylamino)-phénol, le N-cyclopentyl-3-aminophénol, le 1,3-dihydroxy-5-(méthylamino)-benzène, le 3-éthylamino-4-méthylphénol et le 2,4-dichloro-3-aminophénol, comme agent de couplage.

2. Procédé selon la revendication 1, **caractérisé en ce que** l'agent formant une nuance contient en outre au moins un composé peroxo.

3. Procédé selon l'une quelconque des revendications 1 ou 2, **caractérisé en ce que** l'agent formant une nuance est appliqué au moyen d'un applicateur choisi dans le groupe formé par une brosse, un pinceau et une applicette, sur une partie des fibres kératiniques teintées au préalable.

4. Procédé selon l'une quelconque des revendications 1 à 3, **caractérisé en ce que** l'agent formant une nuance présente une viscosité de 5000 à 100 000 mPa.s (viscosimètre rotatif Brookfield, 25°C, mobile #4, 20 t/min).

5. Procédé selon l'une quelconque des revendications 1 à 4, **caractérisé en ce qu'**avant l'étape A, les fibres kératiniques sont recouvertes par un bonnet contenant une grille à trous, le cas échéant uniquement tracée, des paquets de fibres sélectionnés sont tirés au travers des trous, puis l'étape A a lieu et le bonnet est ensuite à nouveau enlevé.

6. Procédé selon l'une quelconque des revendications 1 à 5, **caractérisé en ce qu'**il s'écoule au maximum 60 minutes entre la fin de l'étape A et le début de l'étape C.

7. Procédé selon l'une quelconque des revendications 1 à 6, **caractérisé en ce que** l'agent de teinture est exempt de colorants montant directement sur les fibres.

8. Kit contenant
• le cas échéant un applicateur,
• un récipient C1, contenant un agent de teinture,
• un récipient C2a, contenant une composition contenant du peroxyde d'hydrogène et
• un récipient C2b, contenant une composition contenant au moins un épaississant et au moins un agent d'alcalinisation,
l'agent de teinture contenant, comme composant colorant, au moins deux précurseurs de colorant par oxydation, **caractérisé en ce que** l'agent de teinture contient une combinaison
(a) d'au moins un dérivé de p-phénylènediamine selon la formule (E1), 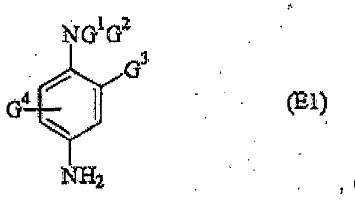 où
- G¹ représente un atome d'hydrogène, un résidu C₁-C₄-alkyle, un résidu C₁-C₄-monohydroxyalkyle, un résidu C₂-C₄-polyhydroxyalkyle, un résidu (C₁-C₄)-alcoxy-(C₁-C₄)-alkyle, un résidu 4'-aminophényle ou un résidu C₁-C₄-alkyle, qui est substitué par un groupe contenant azote, un résidu phényle ou un résidu 4'-aminophényle ;
- G² représente un atome d'hydrogène, un résidu C₁-C₄-alkyle, un résidu C₁-C₄-monohydroxyalkyle, un résidu C₂-C₄-polyhydroxyalkyle, un résidu (C₁-C₄)-alcoxy-(C₁-C₄)-alkyle ou un résidu C₁-C₄-alkyle, qui est substitué par un groupe contenant azote ;
- G³ représente un atome d'hydrogène, un atome d'halogène, tel qu'un atome de chlore, de brome, d'iode ou de fluor, un résidu C₁-C₄-alkyle, un résidu C₁-C₄-monohydroxyalkyle, un résidu C₂-C₄-polyhydroxyalkyle, un résidu C₁-C₄-hydroxyalcoxy, un résidu C₁-C₄-acétylaminoalcoxy, un résidu C₁-C₄-mésylaminoalcoxy ou un résidu C₁-C₄-carbamoylaminoalcoxy ;
- G⁴ représente un atome d'hydrogène, un atome d'halogène ou un résidu C₁-C₄-alkyle ou
- lorsque G³ et G⁴ sont en position ortho l'un par rapport à l'autre, ils peuvent former ensemble un groupe α,ω-alkylènedioxo formant un pont,
(b) d'au moins un dérivé de p-aminophénol selon la formule (E3), 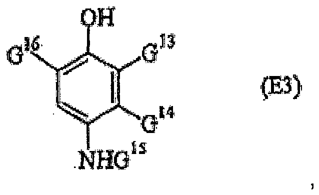 où
- G¹³ représente un atome d'hydrogène, un atome d'halogène, un résidu C₁-C₄-alkyle, un résidu C₁-C₄-monohydroxyalkyle, un résidu C₂-C₄-polyhydroxyalkyle, un résidu (C₁-C₄)-alcoxy-(C₁-C₄)-alkyle, un résidu C₁-C₄-aminoalkyle, un résidu hydroxy-(C₁-C₄)-alkylamino, un résidu C₁-C₄-hydroxyalcoxy, un résidu C₁-C₄-hydroxyalkyl-(C₁-C₄)-aminoalkyle ou un résidu (di-C₁-C₄-alkylamino)-(C₁-C₄)-alkyle, et
- G¹⁴ représente un atome d'hydrogène ou d'halogène, un résidu C₁-C₄-alkyle, un résidu C₁-C₄-monohydroxyalkyle, un résidu C₂-C₄-polyhydroxyalkyle, un résidu (C₁-C₄)-alcoxy-(C₁-C₄)-alkyle, un résidu C₁-C₄-aminoalkyle ou un résidu C₁-C₄-cyanoalkyle,
- G¹⁵ représente hydrogène, un résidu C₁-C₄-alkyle, un résidu C₁-C₄-monohydroxyalkyle, un résidu C₂-C₄-polyhydroxyalkyle, un résidu phényle ou un résidu benzyle, et
- G¹⁶ représente hydrogène ou un atome d'halogène,
(c) d'au moins un dérivé de pyridine, choisi parmi la 2,6-dihydroxypyridine, la 2-amino-3-hydroxypyridine, la 2-amino-5-chloro-3-hydroxypyridine, la 3-amino-2-méthylamino-6-méthoxypyridine, la 2,6-dihydroxy-3,4-diméthylpyridine, la 2,6-dihydroxy-4-méthylpyridine, la 2,6-diaminopyridine, la 2,3-diamino-6-méthoxypyridine et la 3,5-diamino-2,6-diméthoxypyridine, comme agent de couplage, ainsi que
(d) d'au moins un composé choisi parmi le m-aminophénol, le 5-amino-2-méthylphénol, le N-cyclopentyl-3-aminophénol, le 3-amino-2-chloro-6-méthylphénol, le 2-hydroxy-4-aminophénoxyéthanol, le 2,6-diméthyl-3-aminophénol, le 3-trifluoroacétylamino-2-chloro-6-méthylphénol, le 5-amino-4-chloro-2-méthylphénol, le 5-amino-4-méthoxy-2-méthylphénol, le 5-(2'-hydroxyéthyl)amino-2-méthylphénol, le 3-(diéthylamino)-phénol, le N-cyclopentyl-3-aminophénol, le 1,3-dihydroxy-5-(méthylamino)-benzène, le 3-éthylamino-4-méthylphénol et le 2,4-dichloro-3-aminophénol, comme agent de couplage.

9. Kit selon la revendication 8, **caractérisé en ce que** l'agent formant une nuance résultant du mélange du contenu des récipients C2a et C2b présente une viscosité de 5000 à 100 000 mPa.s (viscosimètre rotatif Brookfield, 25°C, mobile #4, 20 t/min).

10. Kit selon la revendication 8 ou 9, caractérisé en que les précurseurs de colorant par oxydation dans un récipient C1a et une composition contenant du peroxyde d'hydrogène dans un récipient C1b sont confectionnés séparément.

11. Utilisation du kit selon l'une quelconque des revendications 8 à 10 dans un procédé pour la teinture de fibres kératiniques selon l'une quelconque des revendications 1 à 7.
